(19) Europäisches Patentamt
European Patent Office
Office européen des brevets

(11) **EP 3 307 304 B1**

(12) **EUROPEAN PATENT SPECIFICATION**

(45) Date of publication and mention
of the grant of the patent:
**08.05.2024 Bulletin 2024/19**

(51) International Patent Classification (IPC):
**A61K 39/00** (2006.01)  **A61K 39/395** (2006.01)
**C07K 16/40** (2006.01)  **C07K 14/535** (2006.01)
**C07K 14/565** (2006.01)  **C07K 19/00** (2006.01)

(21) Application number: **16808386.3**

(22) Date of filing: **10.06.2016**

(52) Cooperative Patent Classification (CPC):
**C07K 14/565; C07K 14/505; C07K 14/535;
C07K 14/56; C12N 9/644;** C07K 2317/53;
C07K 2319/30

(86) International application number:
**PCT/US2016/036915**

(87) International publication number:
**WO 2016/201244 (15.12.2016 Gazette 2016/50)**

(54) **IMMUNOGLOBULIN FUSION PROTEINS AND USES THEREOF**

IMMUNGLOBULIN-FUSIONSPROTEINE UND VERWENDUNGEN DAVON

PROTÉINES DE FUSION À BASE D'IMMUNOGLOBULINES ET LEURS UTILISATIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.06.2015 US 201562175186 P
20.01.2016 US 201615002396
09.06.2016 US 201615177605**

(43) Date of publication of application:
**18.04.2018 Bulletin 2018/16**

(73) Proprietor: **UBI Pharma Inc.
Hsinchu County 30351 (TW)**

(72) Inventors:
• **WANG, Chang-Yi
Cold Spring Harbor, New York 11724 (US)**
• **PENG, Wen-Jiun
Hukou Township
Hsinchu County 30351 (TW)**
• **KAO, Wei-Ting
Hukou Township
Hsinchu County 30351 (TW)**

(74) Representative: **Weickmann & Weickmann
PartmbB
Postfach 860 820
81635 München (DE)**

(56) References cited:
WO-A1-2006/074199  WO-A1-2008/012543
WO-A1-2011/063348  WO-A1-2012/006633
WO-A2-2004/042017  WO-A2-2008/143954
US-A1- 2005 142 642  US-A1- 2009 092 607
US-A1- 2011 081 345  US-A1- 2012 100 099
US-A1- 2013 281 677

• BEALS JM, SHANAFELT AB: "Enhancing
exposure of protein therapeutics", DRUG
DISCOVERY TODAY: TECHNOLO, ELSEVIER,
AMSTERDAM, NL, vol. 3, no. 1, 1 April 2006
(2006-04-01), pages 87-94, XP005409090, ISSN:
1740-6749, DOI: 10.1016/J.DDTEC.2006.03.001
• DAVIS PATRICIA M ET AL: "Abatacept binds to
the fc receptor CD64 but does not mediate
complement-dependent cytotoxicity or
antibody-dependent cellular cytotoxicity",
JOURNAL OF RHEUMATO, JOURNAL OF
RHEUMATOLOGY PUBLISHING COMPANY, CA,
vol. 34, no. 11, 1 November 2007 (2007-11-01),
pages 2204-2210, XP009143439, ISSN: 0315-162X
• RATH ET AL.: 'Fc-fusion proteins and FcRn:
structural insights for longer-lasting and more
effective therapeutics' CRIT. REV. BIOTECHNOL.
vol. 35, no. 2, 24 October 2013, pages 235 - 254,
XP002753804

EP 3 307 304 B1

Remarks:
The complete document including Reference Table(s) and the Sequence Listing(s) can be downloaded from the EPO website

**Description**

**FIELD OF THE INVENTION**

The present invention relates to a therapeutic fusion protein comprising an Fc fragment of an immunoglobulin G and a bioactive molecule, wherein the Fc fragment is a single chain.

**BACKGROUND OF THE INVENTION**

An immunoglobulin comprises four polypeptide chains, two heavy chains and two light chains, that associate via interchain disulfide bonds. Each light chain has two domains, a variable light domain ($V_L$) and a constant light domain ($C_L$); and each heavy chain has two regions, a variable heavy region ($V_H$) and a constant heavy region ($C_H$). The constant heavy region ($C_H$) is composed of constant heavy domains that are designated by number (e.g., $C_H1$, $C_H2$, $C_H3$, etc.) (*see e.g.,* US 6,086,875 (Blumberg R. S. et al.); US 5,624,821 (Winter G. P. et al.); and US 5,116,964 (Capon D. J. and Lasky L . A.)). Immunoglobulins are categorized into different isotypes based on their biological properties, location in an organism, and ability to deal with different antigens (*i.e.,* IgG, IgM, IgA, IgD and IgE). Depending on the immunoglobulin isotype, the constant heavy region ($C_H$) can have three or four $C_H$ domains. Also, in some isotypes (IgA, IgD, and IgG), the heavy chains contain a hinge region that adds flexibility to the molecule (Janeway et al. 2001, Immunobiology, Garland Publishing, N.Y., N.Y.).

There are four IgG subclasses (IgG1, 2, 3, and 4) in humans, named in order of their abundance in serum (IgG1 being the most abundant). The IgG isotype, is composed of two light chains and two heavy chains, where each heavy chain contains three constant heavy domains ($C_H1$, $C_H2$, $C_H3$). The two heavy chains of IgG are linked to each other and to a light chain each by disulfide bonds (-S-S-). The antigen binding site of IgG is located in the Fragment antigen binding region (Fab region), which contains variable light ($V_L$) and variable heavy ($V_H$) chain domains as well as constant light ($C_L$) and constant heavy ($C_H1$) chain domains. The fragment crystallizable region (Fc region) of IgG is a portion of the heavy chain containing the $C_H2$ and $C_H3$ domains that binds to an Fc receptor found on the surface of certain cells, including the neonatal Fc receptor (FcRn). The heavy chain of IgG also has a hinge region (hinge) between the $C_H1$ and $C_H2$ domains that separates the Fab region from the Fc region and participates in linking the two heavy chains together via disulfide bonds. The structure of the hinge region contributes to unique biological properties of each of the four IgG subclasses.

IgG is secreted as a monomer that is small in size allowing it to easily perfuse tissues. It is the only isotype that has receptors (neonatal Fc receptor (FcRn)) that facilitate passage through the human placenta to provide protection to the fetus *in utero.* IgG absorbed through the placenta provides the neonate with humoral immunity before its own immune system develops.

The IgG neonatal Fc receptor (FcRn) binding site is located in the Fc region of the antibody. FcRn is normally expressed in human placenta and epithelial cells and participates in an endocytic salvage pathway that prevents degradation of IgG. This salvage pathway is mediated by the highly pH-dependent binding affinity of IgG to FcRn in acidic pH. The high affinity of IgG for FcRn at acidic pH is believed to result in binding of internalized IgG to FcRn after uptake into acidic endosomes (Goebl NA, et al, 2008; Junghans RP, et al, 1996). Although most soluble proteins are directed to lysosomes after internalization, internalized FcRn-bound IgG returns to the plasma membrane and is effectively rescued from the default degradative pathway. Upon exposure to the neutral pH of the extracellular space, IgG can then dissociate from FcRn and return to circulation. Thus, the extended serum half-life property of the antibody is retained in the Fc fragment.

This salvage pathway provides one mechanism for developing next-generation protein drugs that have a prolonged half-life in blood circulation compared to unmodified protein drugs. In particular, unmodified protein drugs have a short circulating half-life, making frequent dosing over an extended treatment period necessary. Extensive efforts have been made to extend the half-life of the protein drugs by many means including PEGylation fusion protein technologies (U.S. Food and Drug Administration; Osborn BL, et al, 2002); however, the results from these efforts have not been ideal.

The creation of fusion proteins comprising IgG constant regions linked to a protein of interest, or fragment thereof, has been described. For example, protein "X" of interest is linked to an IgG "Fc" domain to create an "Fc-X" or "X-Fc" fusion protein (immunofusion). Immunofusion proteins can generally be prepared and purified in larger quantities compared to other types of fusion proteins because the Fc moiety of the fusion protein is designed for efficient secretion by the cell. Fusion proteins containing a Fc region of an immunoglobulin have been shown to have enhanced features compared to their non-Fc-containing counterparts, including increased protein stability and longer serum half-life (*see* Capon et al. 1989, Nature 337:525), as well as an ability to bind to Fc receptors such as the neonatal Fc receptor (FcRn) (*see e.g.,* US 6,086,875 (Blumberg R. S. et al.); US 6,485,726 (Blumberg R. S. et al.); US 6,030,613; WO 03/077834 (Blumberg R. S. et al.); and US 2003-0235536A1 (Blumberg R. S. et al.)). The following patent documents describe additional examples.

US 5,116,964 (Capon D. J. and Lasky L. A.) discloses a ligand binding partner protein which comprises a lymphocyte cell surface glycoprotein (LHR) and an immunoglobulin chain, in which the ligand binding partner protein and immunoglobulin are fused through either N-terminus amino or C-terminus carboxyl group.

WO 94/04689 (Pastan I. H. et al.) discloses the use of Fc of immunoglobulin to provide a toxin with extended half-life that includes a ligand binding domain (CD4 receptor) and a *Pseudomonas* exotoxin A. The IgG Fc links the CD4 receptor and a *Pseudomonas* exotoxin A.

US 6,797,493 (Sun L-H. et al.) discloses a hG-CSF-L-vFc fusion protein comprising human granulocyte colony-stimulating factor (hG-CSF), a flexible peptide linker (L) of about 20 or fewer amino acids, and a human IgG Fc variant. The Fc variant is of a non-lytic nature and shows minimal undesirable Fc-mediated side effects.

US 8,557,232 (Gillies S. D. et al.) discloses a method and composition for expressing soluble, biologically active Fc-IFN-$\beta$ fusion proteins and variants thereof (Fc-IFN-$\beta^{sol}$). The Fc-IFN-$\beta$ fusion protein includes an IFN-$\beta$ protein linked to the carboxy-terminus of the immunoglobulin Fc region

The above documents describe fusion proteins having a two chain Fc fusion protein design with two bioactive molecules in close proximity to one another. The bioactive molecules of these traditional fusion proteins are generally suppressed or sterically hindered from interacting with the target molecules or cells. Therefore, there is a need to develop a fusion protein comprising a bioactive molecule linked to a modified Fc region of an IgG, that can confer increased *in vivo* half-life of the bioactive molecules without suppressing the bioactivity of the bioactive molecule. Such modified fusion proteins would provide an added benefit for ease of purification by related affinity purification processes.

**References:**

1. Blumberg R. S. et al., "Receptor specific transepithelialus transport of therapeutics" US Patent Nos. 6,030,613 (2000), 6,086,875 (2000), and 6,485,726 (2002)

2. Blumberg R. S. et al., "Central airway administration for systemic delivery of therapeutics" WO 03/077834 (2002) and US Patent Application 2003-0235536A1 (2003)

3. Capon D. J., et al., "Designing CD4 immunoadhesins for AIDS therapy" Nature 337:525 (1989)

4. Capon D. J. and Lasky L. A., "Hybrid immunoglobulins" US Patent 5,116,964 (1992)

5. Gillies S. D. et al., "Stabilization of Fc-interferon-beta fusion proteins" US Patent 8,557,232 (2013)

6. Goebl N.A., et al, "Neonatal Fc Receptor Mediates Internalization of Fc in Transfected Human Endothelial Cells" Mol. Biol. Cell, 19(12): 5490-5505 (2008)

7. Janeway et al., Immunobiology, Garland Publishing, N.Y., N.Y (2001)

8. Junghans R.P, et al., "The protection receptor for IgG catabolism is the beta2-microglobulin-containing neonatal intestinal transport receptor" Proc Natl Acad Sci USA.93(11): 5512-5516 (1996)

9. Ober R. J. et al, Exocytosis of IgG as mediated by the receptor, FcRn: an analysis at the single-molecule level. Proc Natl Acad Sci USA. 101(30):11076-81 (2004)

10. Ober R. J. et al., Visualizing the site and dynamics of IgG salvage by the MHC class I-related receptor, FcRn. J Immunol. 172(4):2021-9 (2004)

11. Osborn B. L. et al., "Pharmacokinetic and pharmacodynamic studies of a human serum albumin-interferon-alpha fusion protein in cynomolgus monkeys." J Pharmacol Exp Ther.303(2):540-8 (2002)

12. Pastan I.H., et al, "Recombinant toxin with increased half-life" WO 94/04689 (1993)

13. Peters R. T. et al., Prolonged activity of factor IX as a monomeric Fc fusion protein. Blood. 115(10):2057-64 (2010)

14. Sun L-H. et al., "Fc fusion proteins of human granulocyte colony-stimulating factor with increased biological activities" US Patent 6,797,493 (2004)

15. Winter G P. et al., "Antibodies with altered effector functions" US Patent 5,624,821 (1997)

16. Vaccaro C, et al., Engineering the Fc region of immunoglobulin G to modulate in vivo antibody levels. Nat Biotechnol.23(10):1283-8 (2005)

**SUMMARY OF THE INVENTION**

The present disclosure is directed to novel fusion proteins comprising a portion of an immunoglobulin (Ig) molecule, compositions thereof, and methods for making the disclosed fusion proteins. The disclosed fusion proteins are useful for extending the serum half-life of bioactive molecules in an organism.

One aspect of the present disclosure relates to a fusion protein produced in a mammalian cell, and compositions thereof. The fusion protein of the present disclosure generally comprises (a) bioactive molecule selected from the group consisting of erythropoietin, Factor IX, IFN$\alpha$, GCSF, and IFN$\beta$ and (b) a single chain Fc (sFc) fragment of an IgG molecule having an amino acid sequence selected from the group consisting of SEQ ID NOs: 61, 62, 63, and 64, and c) a hinge region between the bioactive molecule and the sFc fragment, wherein the hinge region is mutated and does not form disulfide bonds with another fusion protein or another immunoglobulin molecule, and wherein the hinge region comprises an amino acid sequence selected from the group consisting of SEQ ID NOs: 23-60. The amino acid sequence of the (a) bioactive molecule and (b) Ig fragment of the fusion protein are derived from the wild-type sequence of each fragment, as disclosed in the art. Derived sequences include the wild-type sequence as well as homologues, analogues, fragments, and other variants of the wild-type sequence.

The Ig fragment of the fusion protein comprises a single chain Fc (sFc or scFc), a monomer, that is incapable of forming a disulfide-bonded dimer. The fusion protein further includes a sequence corresponding to an immunoglobulin hinge region. The hinge region contains a modification that prevents the fusion protein from forming a disulfide bond with another fusion protein or another immunoglobulin molecule. The hinge region is modified by mutating and/or deleting one or more cysteine amino acids to prevent the formation of a disulfide bond. The bioactive molecule is linked to the scFc through a hinge region. In specific embodiments, the fusion protein comprises the bioactive molecule at its N-terminus that is linked to a scFc through a mutated hinge region. In certain embodiments, the present invention relates to compositions, including pharmaceutical compositions, comprising the fusion protein and a pharmaceutically acceptable carrier or excipient.

Another aspect of the present invention relates to methods for making a fusion protein and compositions thereof. The method for making the fusion protein is carried out in a mammalian cell and comprises (i) providing a bioactive molecule and an sFc fragment comprising a hinge region, wherein the bioactive molecule selected from the group consisting of erythropoietin, Factor IX, IFN$\alpha$, GCSF, and IFN$\beta$, (ii) mutating the hinge region of the sFc fragment by amino acid substitution and/or deletion to form a mutated hinge region of a mutated sFc fragment, and (iii) linking the bioactive molecule to the Fc fragment through the mutated hinge region to form the fusion protein, or composition thereof, wherein the mutated hinge region comprises an amino acid sequence selected from the group consisting of SEQ ID NOs: 23-60, and wherein the mutated hinge region does not form disulfide bonds with another fusion protein or another immunoglobulin molecule.

Detailed description of the invention is given in the following embodiments with reference to the accompanying drawings.

## BRIEF DESCRIPTION OF THE DRAWINGS

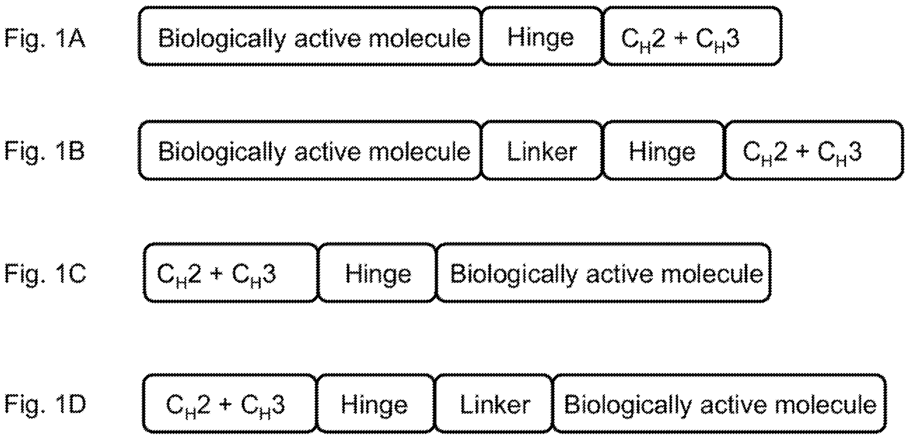

Figures 1A to 1D illustrate the design of a single chain fusion protein according to various embodiments of the present disclosure. Fig. 1A illustrates a fusion protein comprising a biologically active molecule at the N-terminus that is covalently linked to a hinge region and Fc fragment ($C_H2$ and $C_H3$ domains) of human IgG. Fig. 1B illustrates a fusion protein comprising a biologically active molecule at the N-terminus that is covalently linked to a hinge region and Fc fragment ($C_H2$ and $C_H3$ domains) of human IgG through a linker. Fig. 1C illustrates a fusion protein comprising a biologically active molecule at the C-terminus that is covalently linked to a hinge region and Fc fragment ($C_H2$ and $C_H3$ domains) of human IgG Fig. 1D illustrates a fusion protein comprising a biologically active molecule at the C-terminus that is covalently linked to a hinge region and Fc fragment ($C_H2$ and $C_H3$ domains) of human IgG through a linker.

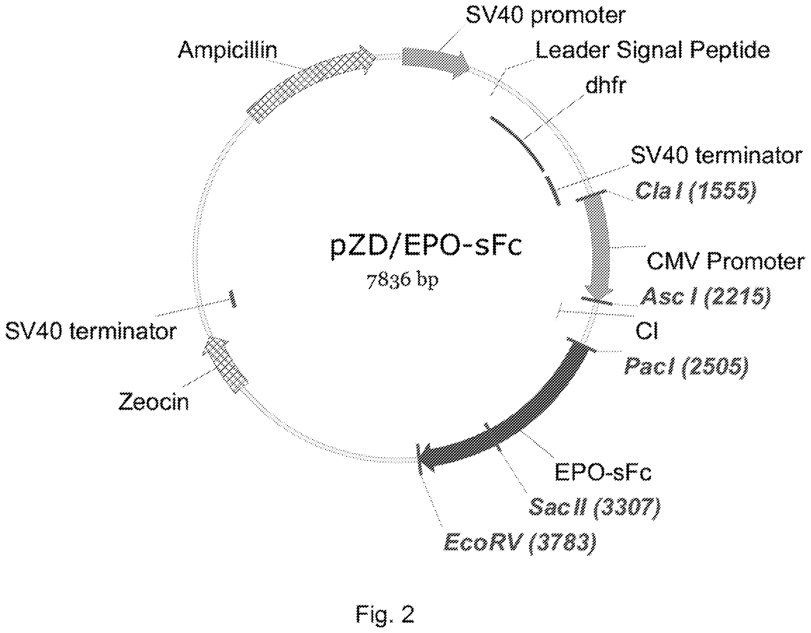

Figure 2 illustrates a map of pZD/EPO-sFc plasmid. The pZD/EPO-sFc plasmid encodes an EPO-sFc fusion protein according to an embodiment of the present invention.

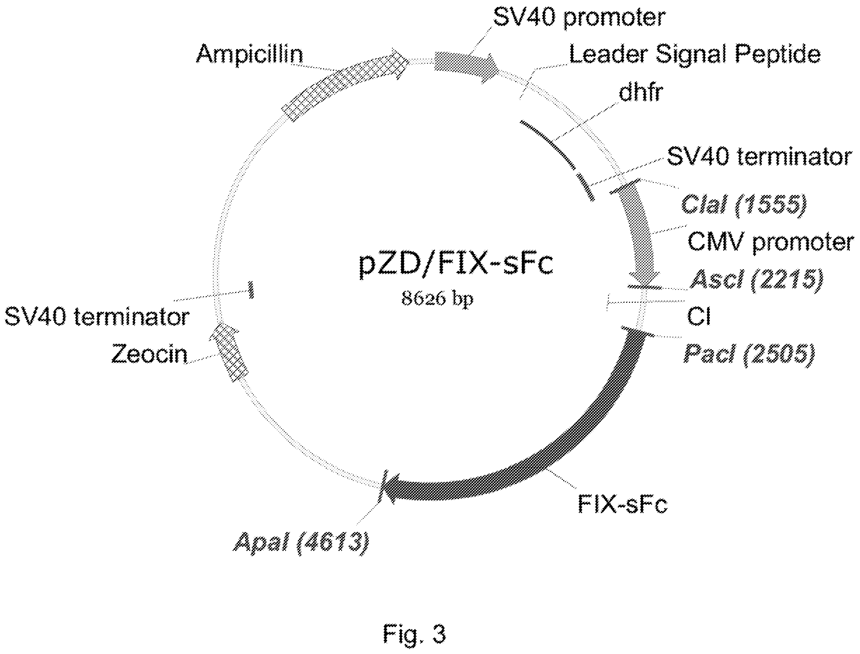

Figure 3 illustrates a map of pZD/FIX-sFC plasmid. The pZD/FIX-sFc plasmid encodes a Factor IX-sFc fusion protein according to an embodiment of the present invention.

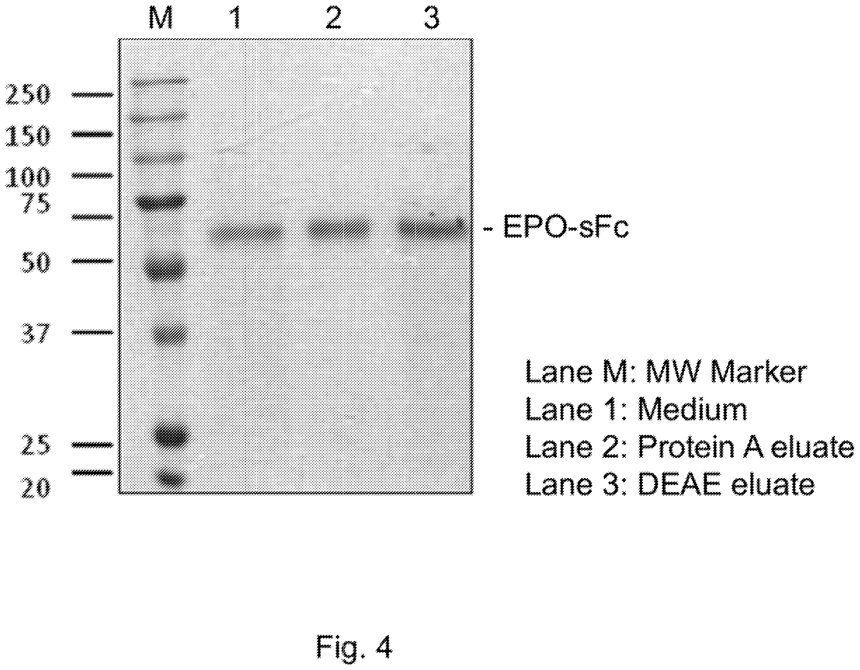

Figure 4 illustrates an SDS-PAGE profile, by Coomassie blue staining, of erythropoietin single chain Fc fusion protein (EPO-sFc) produced by methods disclosed herein. Lane M is a molecular weight marker (marker contains recombinant proteins in size of 20, 25, 37, 50, 75, 100, 150, and 250 kDa). Lane 1 is an EPO-sFc fusion protein in cell culture medium. Lane 2 is an eluate of EPO-sFc fusion protein purified by Protein A resin (MabSelect SuRe™ Hitrap column). Lane 3 is an eluate of EPO-sFc fusion protein purified by DEAE column. The MW of EPO-sFc (Lanes 1 to 3) is between 50-70 KDa with high content of glycosylation.

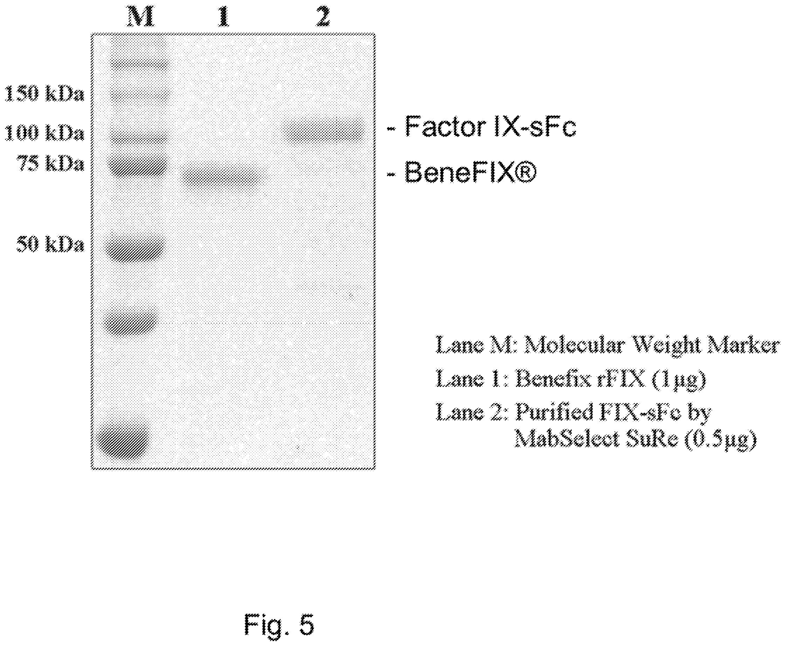

Figure 5 illustrates an SDS-PAGE profile, by Coomassie blue staining, of Factor IX single chain Fc fusion protein (Factor IX-sFc) produced by methods disclosed herein. Lane M is a molecular weight marker (marker contains recombinant protein in size of 50, 75, 100, and 150 kDa). Lane 1 is an original recombinant human Factor IX (BeneFIX®). Lane 2 is an eluate of Factor IX-sFc fusion protein purified by Protein A resin (MabSelect SuRe™ Hitrap column). The MW of Factor IX-sFc (Lane 2) is between 100-110 KDa with high content of glycosylation.

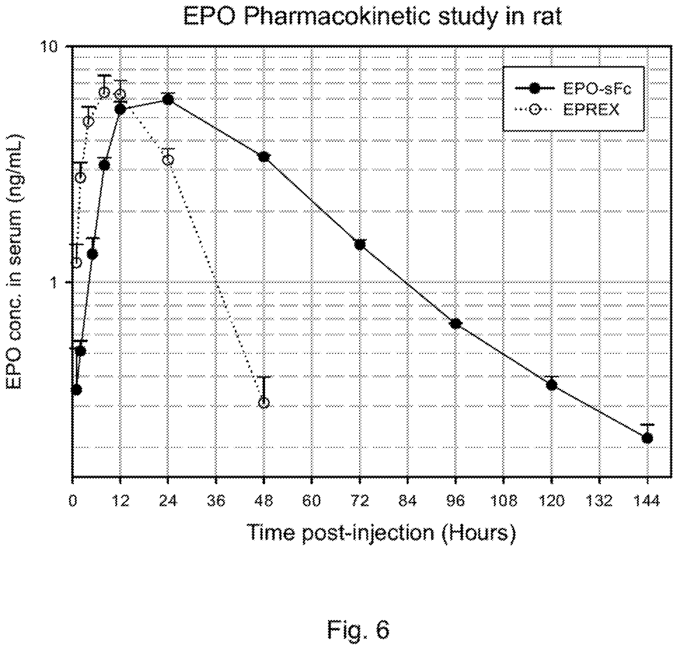

**Figure 6** is a graph showing the pharmacokinetics (PK) profile of a single dose subcutaneous (S.C.) administration of erythropoietin single chain Fc fusion protein (EPO-sFc) (closed circle) and original recombinant human EPO (EPREX®) (open circle) in rats. For single dose S.C. administration of EPO-sFc at 16.8 μg/kg, the half-life of EPO-sFc is about 22 hours, which is 3 times longer than EPREX®.

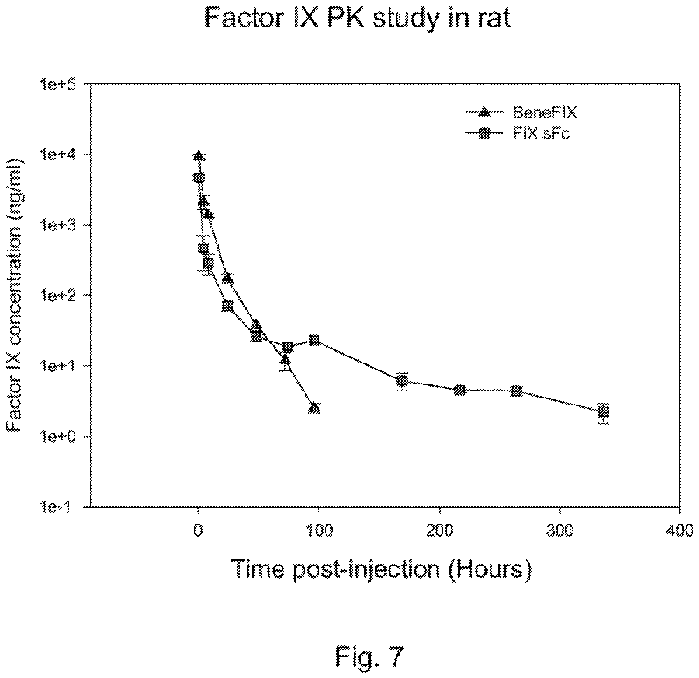

**Figure 7** is a graph showing the pharmacokinetics (PK) profile of a single dose intravenous (I.V.) administration of Factor IX single chain Fc fusion protein (FIX-sFc) (square) and original recombinant human Factor IX (BeneFIX®) (triangle) in rats. The half-life of FIX-sFc is about 56 hours for I.V administration, which is 5 times longer than BeneFIX® ($T_{1/2}$ = 11.91 hours).

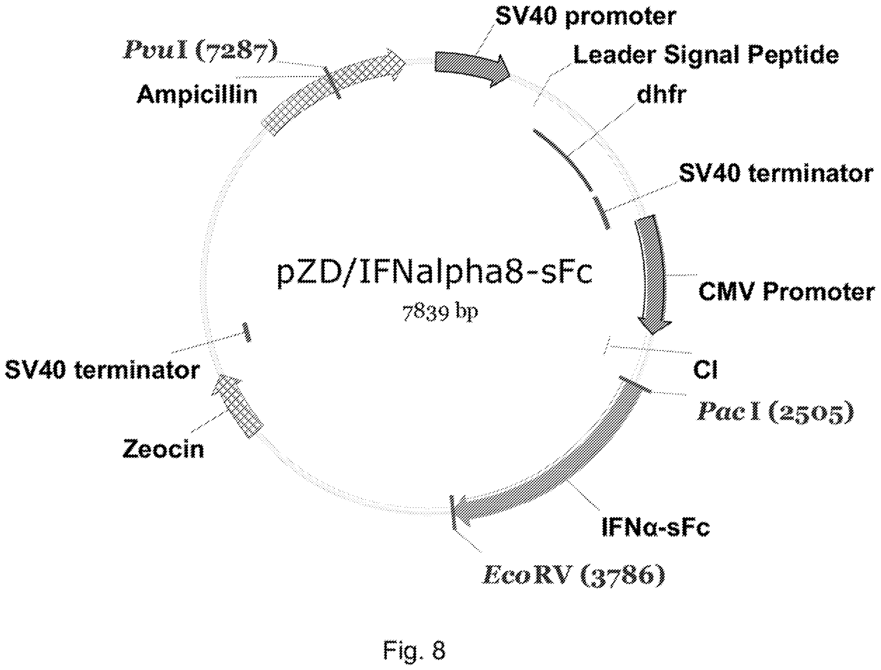

**Figure 8** illustrates a plasmid map of pZD/IFNα-sFc. The pZD/ IFNα-sFc plasmid encodes an IFNα-sFc fusion protein according to an embodiment of the present invention.

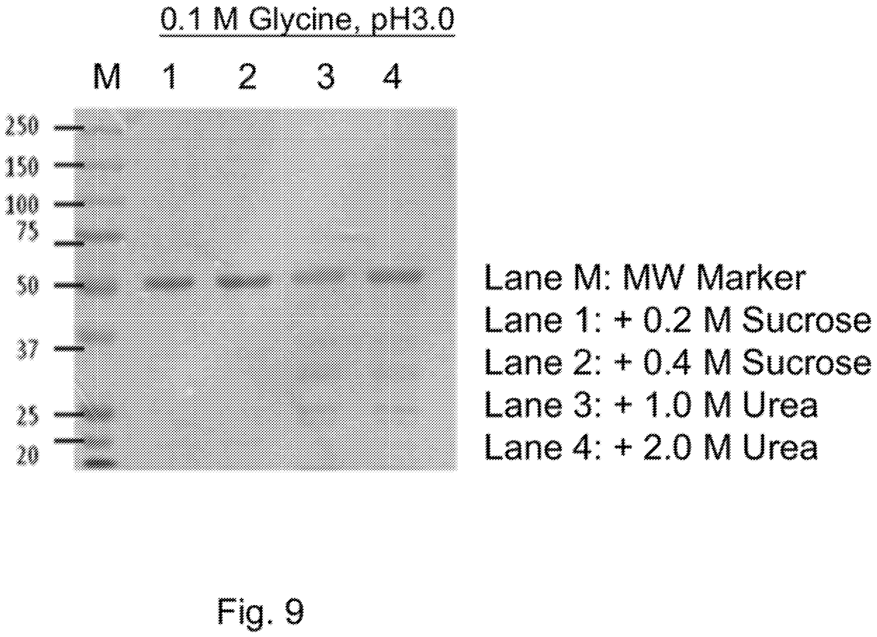

**Figure 9** illustrates an SDS-PAGE profile, by Coomassie blue staining, of Interferon alpha single chain Fc fusion protein (IFNα-sFc) produced by methods disclosed herein. Lane M is a molecular weight marker (marker contains recombinant proteins in size of 20, 25, 37, 50, 75, 100, 150, and 250 kDa). Lanes 1 and 2 are eluates of IFNα-sFc fusion protein containing 0.2 to 0.4 M sucrose, respectively. Lanes 3 and 4 are eluates of IFNα-sFc fusion protein containing 1.0 to 2.0 M urea, respectively.

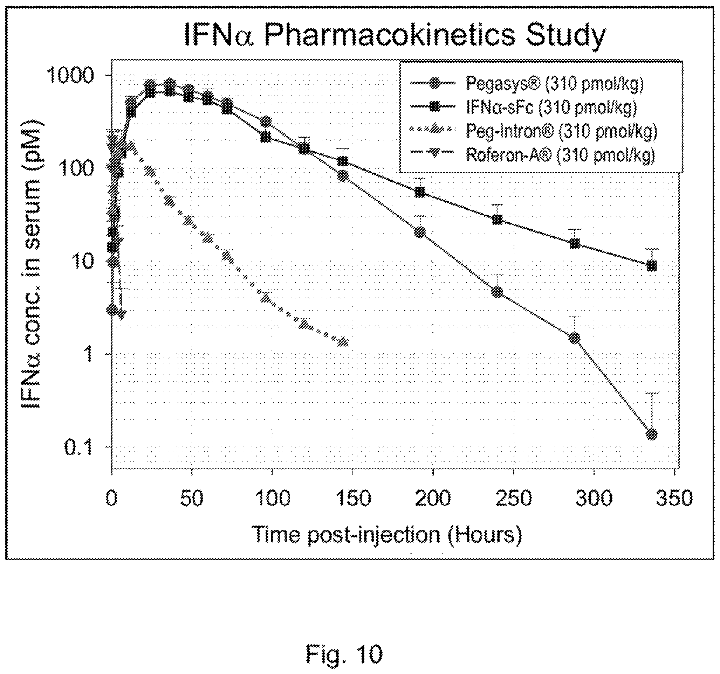

**Figure 10** is a graph showing the pharmacokinetics (PK) profile of a single dose subcutaneous (S.C.) administration of Pegasys® (circle), interferon alpha single chain Fc fusion protein (IFNα-sFc) (rectangle), Peg-Intron® (regular triangle), and Roferon-A® (inverted triangle) in rats. The half-life of IFNα-sFc is about 50.2 hours, which is longer than other interferon products.

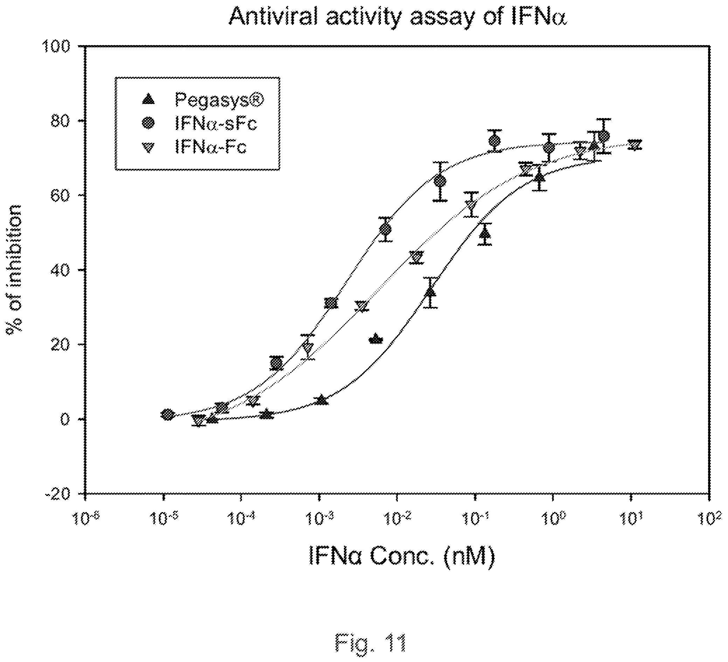

**Figure 11** is a graph showing the anti-virus activity of interferon alpha single chain Fc fusion protein (IFNα-sFc) (circle), Pegasys® (regular triangle) and interferon alpha Fc fusion protein (IFNα-Fc) (inverted triangle) in rats. The antiviral activity ($IC_{50}$) of IFNα-sFc is about 0.0061 nM, which is far better than that of IFNα-Fc ($IC_{50}$ = 0.0313 nM) and Pegasys® ($IC_{50}$ = 0.0894).

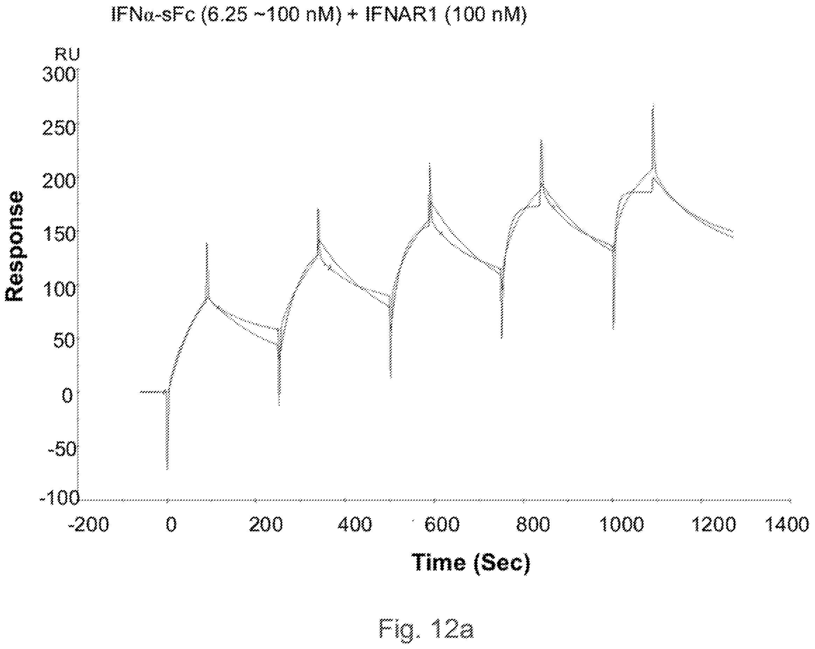

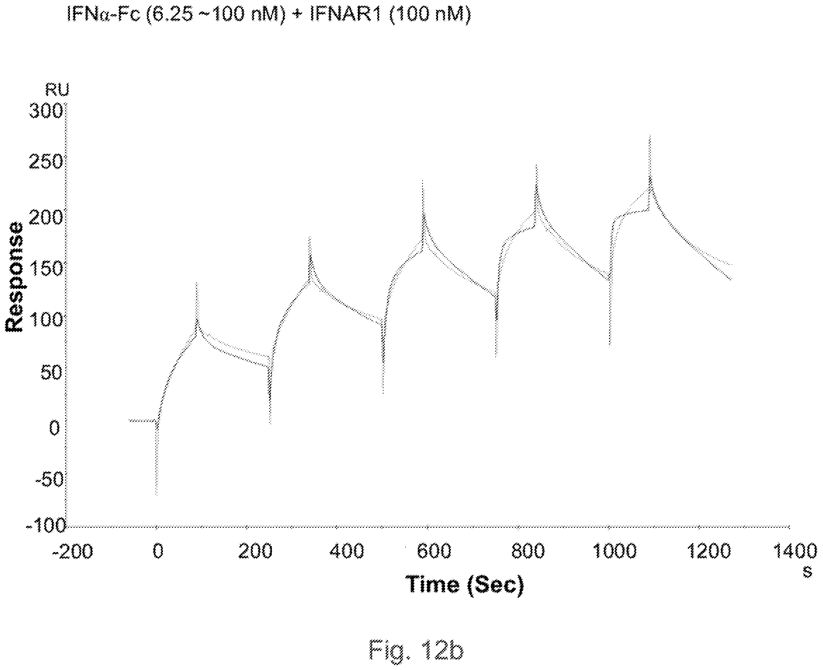

**Figures 12a to 12b** illustrate the association profiles of IFNα-sFc with Interferon-alpha receptor 1 (IFNAR1) **(Figure 12a)** and IFNα-Fc with IFNAR1 **(Figure 12b).**

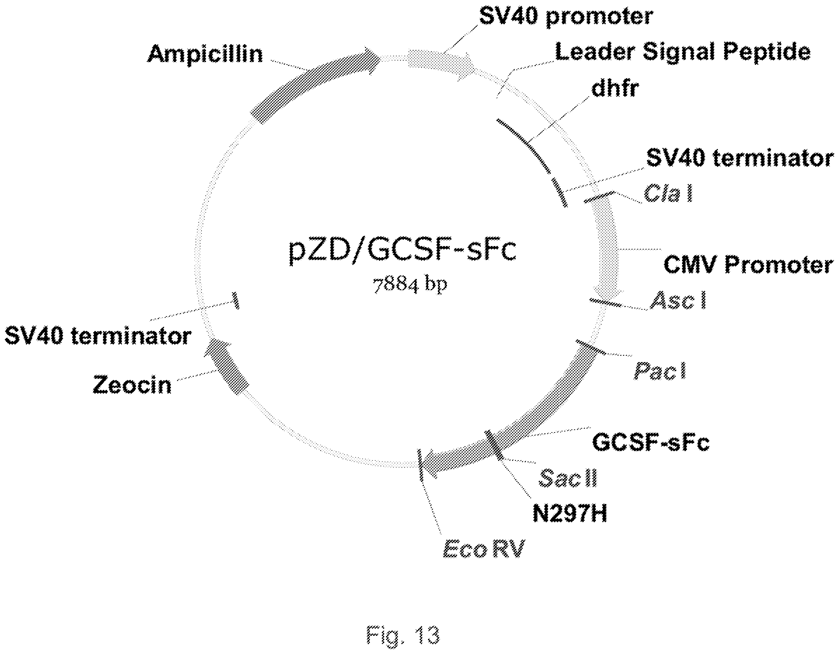

**Figure 13** illustrates a plasmid map of pZD-GCSF-sFc. The pZD/GCSF-sFc plasmid encodes an IFNα8-sFc fusion protein according to an embodiment of the present invention.

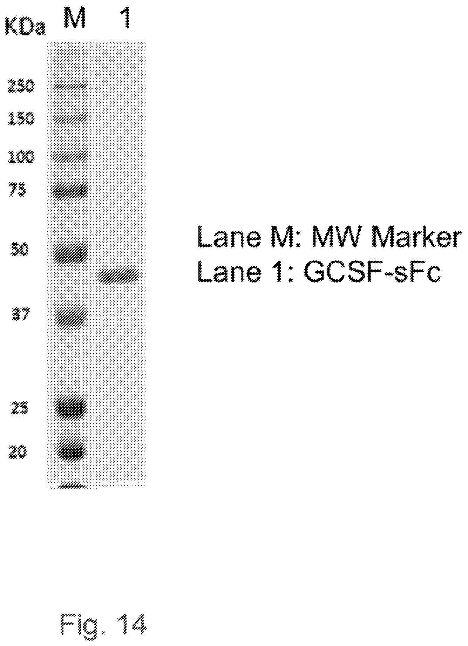

**Figure 14** illustrates an SDS-PAGE profile, by Coomassie blue staining, of GCSF single chain Fc fusion protein (GCSF-sFc) produced by methods disclosed herein. Lane M is a molecular weight marker (marker contains recombinant proteins in size of 20, 25, 37, 50, 75, 100, 150, and 250 kDa). Lane 1 is an eluate of GCSF-sFc.

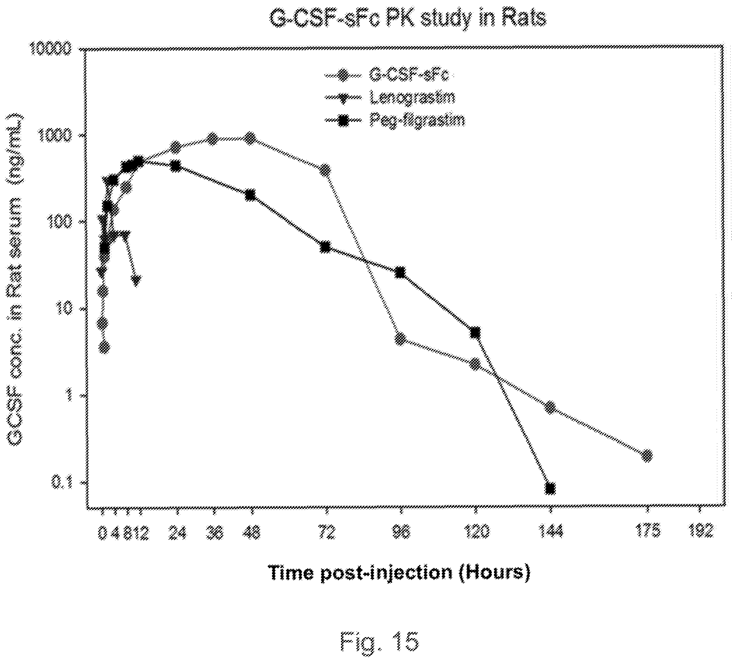

**Figure 15** is a graph showing the pharmacokinetics (PK) profile of a single dose subcutaneous (S.C.) administration of GCSF single chain Fc fusion protein (GCSF-sFc) (circle), Lenograstim (Granocyte®) (triangle), and Peg-filgrastim (Neulasta®) (rectangle) in rats. The half-life of GCSF-sFc is about 17.16 hours, which is longer than that of Lenograstim (4.1 hours) and Peg-filgrastim (12.0 hours).

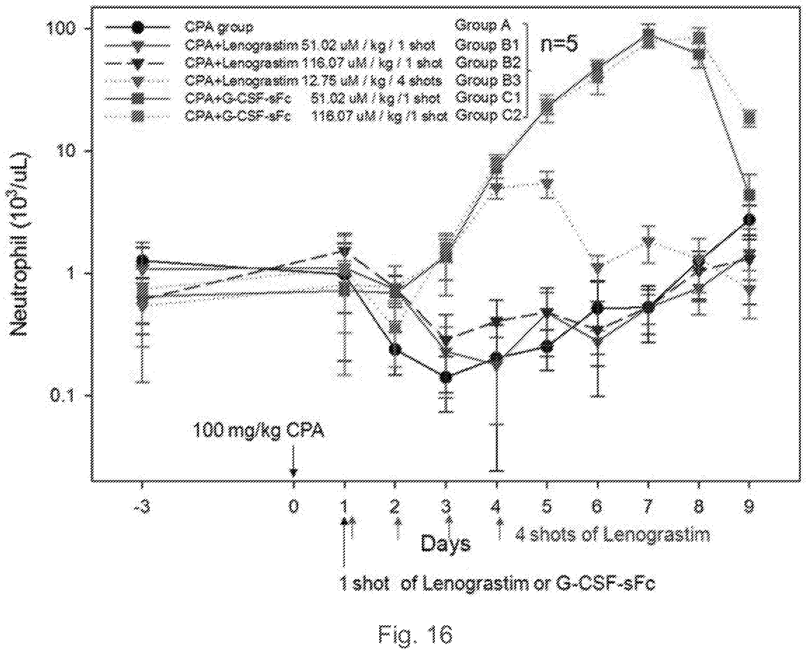

**Figure 16** is a graph showing the biological activity of single dose (groups B1 and B2) or four doses (Group B3) subcutaneous (S.C.) administration of Lenograstim and a single dose subcutaneous (S.C.) administration of GCSF single chain Fc fusion protein (GCSF-sFc) (Groups C1 and C2). The GCSF-sFc has a higher activity to enhance the neutrophil differentiation and proliferation in mice than Lenograstim.

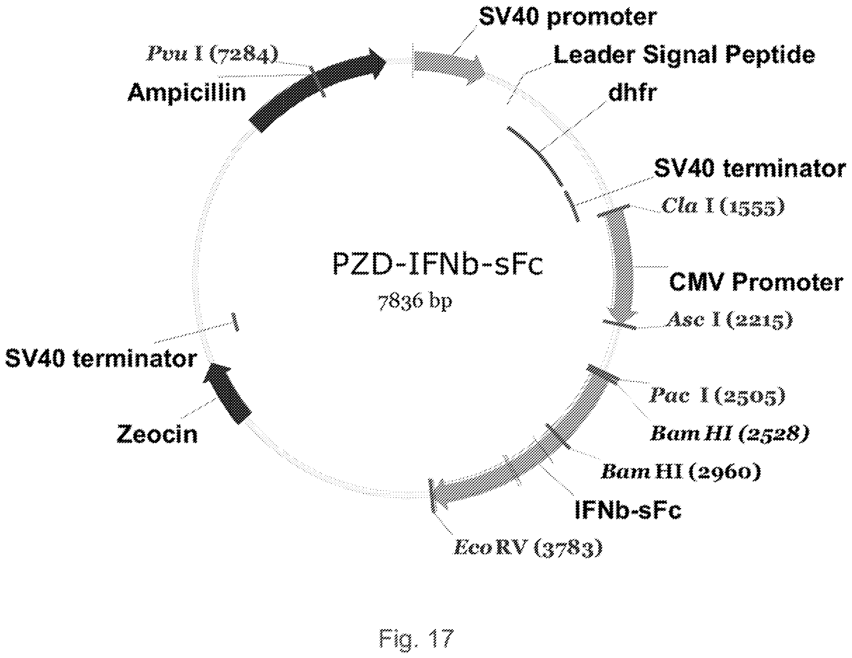

**Figure 17** is a plasmid map of pZD-IFNβ-sFc. The pZD-IFNβ-sFc plasmid encodes an IFNβ-sFc fusion protein according to an embodiment of the present invention.

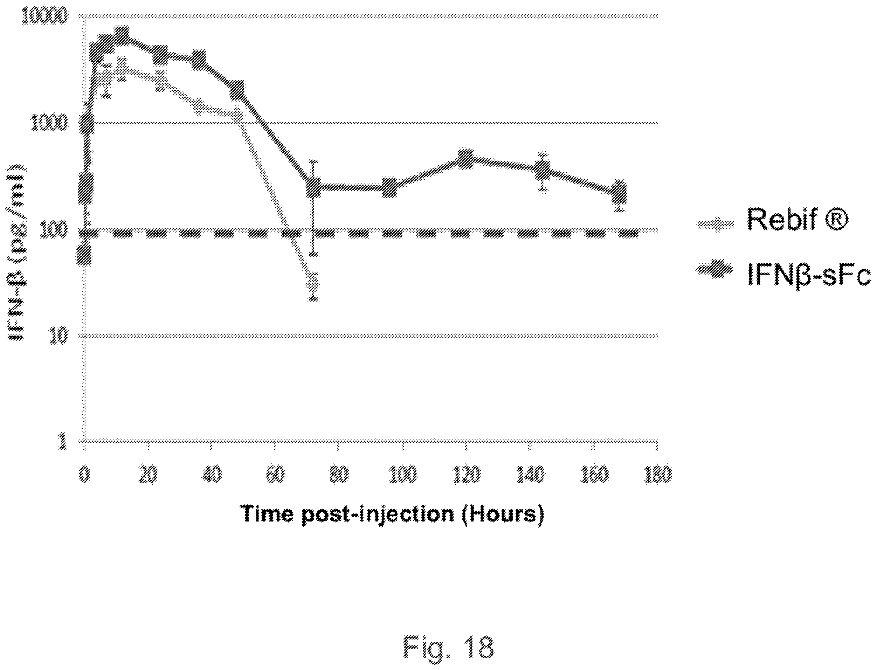

**Figure 18** is a graph showing the pharmacokinetics (PK) profile of a single dose subcutaneous (S.C.) administration of interferon β single chain Fc fusion protein (IFNβ-sFc) (rectangle) and Rebif® (rhombus) in rats. The half-life of IFNβ-sFc is about 31.89-37.7 hours, which is longer than that of Rebif® (18.92-23.57 hours).

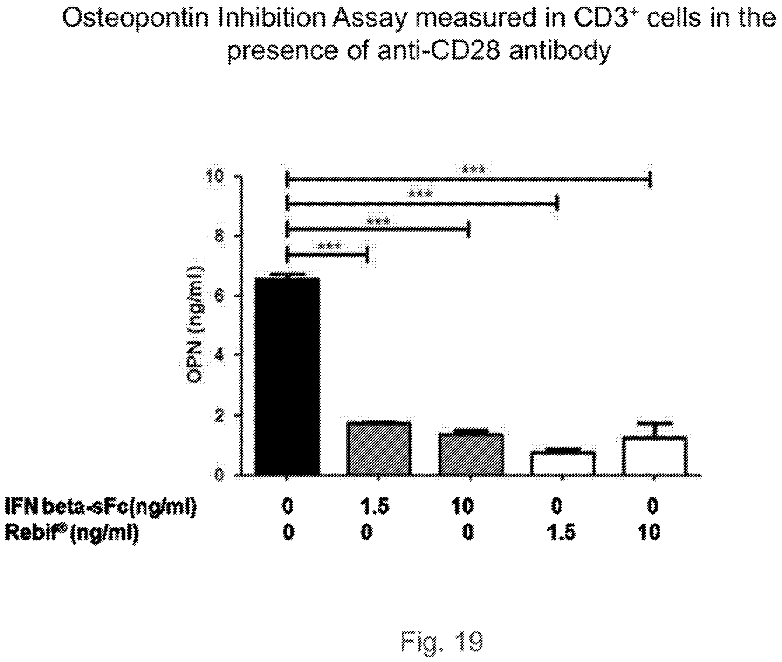

**Figure 19** is a graph showing the osteopontin (OPN) inhibition by IFNβ-sFc fusion protein and Rebif®. The IFNβ-sFc fusion protein exhibited significant inhibition effect of 73.4±0.8% and 79.5±3.6% at both 1.5 ng/mL and 10 ng/mL, respectively. The biological activity of IFNβ-sFc was comparable to Rebif®.

## DETAILED DESCRIPTION OF THE INVENTION

The present disclosure is directed to novel fusion proteins comprising a bioactive molecule and portions of an immunoglobulin molecule. Various aspects of the present disclosure relate to fusion proteins, compositions thereof, and methods for making and using the disclosed fusion proteins. The disclosed fusion proteins are useful for extending the serum half-life of bioactive molecules in an organism.

The following is a detailed description provided to aid those skilled in the art in practicing the present invention. The terminology used in the description is for describing particular embodiments only and is not intended to be limiting of the invention. The section headings used below are for organizational purposes only and are not to be construed as limiting the subject matter described.

### 1. Fusion Protein

As used herein, "fusion protein" or a "fusion polypeptide" is a hybrid protein or polypeptide comprising at least two proteins or peptides linked together in a manner not normally found in nature.

One aspect of the present disclosure is directed to a fusion protein comprising an immunoglobulin (Ig) Fc fragment and a bioactive molecule as defined above. The bioactive molecule that is incorporated into the disclosed fusion protein has improved biological properties compared to the same bioactive molecule that is either not-fused or incorporated into a fusion protein described in the prior art (e.g., fusion proteins containing a two chain Fc region). For example, the bioactive molecule incorporated into the disclosed fusion protein has a longer serum half-life compared to its non-fused counterpart. Additionally, the disclosed fusion protein maintains full biological activity of the bioactive molecule without any functional decrease, which is an improvement over the fusion proteins of the prior art that have a decrease in activity due to steric hindrance from a two chain Fc region.

The fusion proteins of the present disclosure provide significant biological advantages to bioactive molecules compared to non-fused bioactive molecules and bioactive molecules incorporated into fusion proteins described in the prior art.

The disclosed fusion protein can have any of the following formulae (also shown in **Figures 1A to 1D):**

| | | |
|---|---|---|
| (B)-(Hinge)-($C_H2$-$C_H3$) | (Formula 1) | (Fig. 1A) |
| ($C_H3$-$C_H2$)-(Hinge)-(B) | (Formula 2) | (Fig. 1B) |
| (B)-(L)$_m$-(Hinge)-($C_H2$-$C_H3$); or | (Formula 3) | (Fig. 1C) |
| ($C_H3$-$C_H2$)-(Hinge)-(L)$_m$-(B) | (Formula 4) | (Fig. 1D) |

wherein

"B" is a bioactive molecule;
"Hinge" is a hinge region of an IgG molecule;
"$C_H2$-$C_H3$" is the $C_H2$ and $C_H3$ constant region domains of an IgG heavy chain;
"L" is an optional linker; and
"m" may be an any integer or 0.

The various portions/fragments of the fusion protein are discussed further below.

### a. Fc Region and Fc Fragment

The fusion protein of the present disclosure contains an Fc fragment from an immunoglobulin (Ig) molecule, selected from the group consisting of SEQ ID NOs: 61, 62, 63, and 64.

As used below, "Fc region" refers to a portion of an immunoglobulin located in the c-terminus of the heavy chain constant region. The Fc region is the portion of the immunoglobulin that interacts with a cell surface receptor (an Fc receptor) and other proteins of the complement system to assist in activating the immune system. In IgG, IgA and IgD isotypes, the Fc region contains two heavy chain domains ($C_H2$ and $C_H3$ domains). In IgM and IgE isotypes, the Fc region contains three heavy chain constant domains ($C_H2$ to $C_H4$ domains). Although the boundaries of the Fc portion may vary, the human IgG heavy chain Fc portion is usually defined to comprise residues C226 or P230 to its carboxyl-terminus, wherein the numbering is according to the EU index.

In certain embodiments, the fusion protein comprises a $C_H2$-$C_H3$ domain, which is an FcRn binding fragment, that can be recycled into circulation again. Fusion proteins having this domain demonstrate an increase in the *in vivo* half-life of the fusion proteins.

As used herein, "Fc fragment" refers to the portion of the fusion protein that corresponds to an Fc region of an immunoglobulin molecule from any isotype. The Fc fragment comprises the Fc region of IgG The Fc fragment is a single chain Fc (sFc) fragment of an IgG molecule having an amino acid sequence selected from the group consisting of SEQ ID NOs: 61, 62, 63, and 64

The Fc fragment can be obtained or produced by any method known in the art. For example, the Fc fragment can be isolated and purified from an animal, recombinantly expressed, or synthetically produced. In some embodiments, the Fc fragment is encoded in a nucleic acid molecule (e.g., DNA or RNA) and isolated from a cell, germ line, cDNA library, or phage library.

The Fc region and/or Fc fragment includes a hinge region found in some immunoglobulin isotypes (IgA, IgD, and IgG). The Fc fragment is modified by mutating the hinge region so that it does not contain any Cys and cannot form disulfide bonds. The hinge region is discussed further below.

The Fc fragment of the disclosed fusion protein is a single chain Fc. As used herein, "single chain Fc" (of "sFc") means that the Fc fragment is modified in such a manner that prevents it from forming a disulfide-bonded dimer (e.g., by chemical modification or mutation addition, deletion, or substation of an amino acid).

The Fc fragment of the fusion protein is derived from human IgG1, which can include the wild-type human IgG1 amino acid sequence or variations thereof. In some embodiments, the Fc fragment of the fusion protein contains an Asn amino acid that serves as an N-glycosylation site at amino acid position 297 of the native human IgG1 molecule (based on the European numbering system for IgG1, as discussed in U.S. Patent No. 7,501,494), which corresponds to residue 67 in the Fc fragment (SEQ ID NO: 61). In other embodiments, the N-glycosylation site in the Fc fragment is removed by mutating the Asn (N) residue with His (H) (SEQ ID NO: 62) or Ala (A) (SEQ ID NO: 63). An Fc fragment containing a variable position at the N-glycosylation site is shown as SEQ ID NO: 64 in the Sequence Listing.

In some embodiments, the $C_H3$-$C_H2$ domain of the Fc fragment has an amino acid sequence corresponding to the wild-type sequence (disclosed in SEQ ID NO: 61). In certain embodiments, the $C_H3$-$C_H2$ domain of the Fc fragment has the amino acid sequence of SEQ ID NO: 62, where the N-glycosylation site is removed by mutating the Asn (N) residue with His (H). In certain embodiments, the $C_H3$-$C_H2$ domain of the Fc fragment has the amino acid sequence of SEQ ID NO: 63, where the N-glycosylation site is removed by mutating the Asn (N) residue with Ala (A).

## b. Hinge Region

The disclosed fusion protein includes a hinge region found in some immunoglobulin isotypes (IgA, IgD, and IgG). The hinge region separates the Fc region from the Fab region, and adds flexibility to the molecule, and can link two heavy chains via disulfide bonds. Formation of a dimer, comprising two $C_H2$-$C_H3$ domains, is required for the functions provided by intact Fc regions. Interchain disulfide bonds between cysteines in the wild-type hinge region help hold the two chains of the Fc molecules together to create a functional unit.

The hinge region is be derived from IgG, namely IgG1. The hinge region can be a full-length or a modified (truncated) hinge region.

The hinge region contains a modification that prevents the fusion protein from forming a disulfide bond with another fusion protein or an immunoglobulin molecule. The hinge region is modified by mutating and/or deleting one or more cysteine amino acids to prevent the formation of a disulfide bond. The N-terminus or C-terminus of the full-length hinge region may be deleted to form a truncated hinge region. In order to avoid the formation of disulfide bonds, the cysteine (Cys) in the hinge region can be substituted with a non-Cys amino acid or deleted. In specific embodiments, the Cys of hinge region may be substituted with Ser, Gly, Ala, Thr, Leu, Ile, Met or Val. Comparative Examples of wild-type and mutated hinge regions from IgG1 to IgG4 include the amino acid sequences shown in **Table 1** (SEQ ID NOs: 1 to 22). Disulfide bonds cannot be formed between two hinge regions that contain mutated sequences of the inevntion. The IgG1 hinge region of the invention was modified to accommodate various mutated hinge regions with sequences shown in **Table 2** (SEQ ID NOs: 23-60).

## c. Linker

The fusion protein may have the bioactive molecule linked to the N-terminus of the Fc fragment. Alternatively, the fusion protein may have the bioactive molecule linked to the C-terminus of the Fc fragment. The linkage is a covalent bond, and preferably a peptide bond.

In the present invention, one or more bioactive molecule may be directly linked to the C-terminus or N-terminus of the Fc fragment. Preferably, the bioactive molecule(s) can be directly linked to the hinge of the Fc fragment.

Additionally, the fusion protein may optionally comprise at least one linker. Thus, the bioactive molecule may not be directly linked to the Fc fragment. The linker may intervene between the bioactive molecule and the Fc fragment. The linker can be linked to the N-terminus of the Fc fragment or the C-terminus of the Fc fragment.

In one embodiment, the linker includes amino acids. The linker may includes 1-5 amino acids.

### d. Bioactive Molecule

As used herein, the term "biologically active molecule" refers to proteins, glycoproteins, and combinations thereof. The bioactive molecule is selected from the group consisting of erythropoietin, Factor IX, IFNα, GCSF, and IFNβ.

In one embodiment, the biologically active molecule is erythropoietin (EPO). Erythropoietin, an acidic glycoprotein of approximately 34,000 dalton molecular weight, is a glycoprotein hormone involved in the maturation of erythroid progenitor cells into erythrocytes. It is essential in regulating levels of red blood cells in circulation. Naturally occurring erythropoietin is produced by the liver during fetal life and by the kidney of adults and circulates in the blood and stimulates the production of red blood cells in bone marrow. See, Erythropoietin concentrated solution of European Pharmacopoeia. In a specific embodiment, the EPO protein has an amino acid sequence of SEQ ID NO: **65.**

In another embodiment, the biologically active molecule is Factor IX (FIX). FIX, a globular protein which has a molecular weight of about 70,000 daltons, is a vitamin K-dependent protein which participates in blood coagulation. It is synthesized in the form of a zymogen and undergoes three types of post-translational modifications before being secreted into the blood. In man, the liver is the site of FIX synthesis. This protein participates in the blood coagulation cycle and is used for the treatment of hemophilia B patients. At the present time the only commercially available source of FIX is human plasma. See, Human coagulation Factor IX of European Pharmacopoeia. In a specific embodiment, the FIX protein has an amino acid sequence of SEQ ID NO: **67.**

In other embodiments, the biologically active molecule is Interferon alpha (IFNα) or Interferon beta (IFNβ). Interferons (IFNs) are glycoproteins (19-20 KDa), possessing anti-viral, immunomodulatory and anti-proliferative effects and are divided in to three classes (Types I, II and III) according to their structural homology and the specific receptor they associate with. The type I IFN family includes numerous IFN alpha variants, a single IFN beta member, and lesser known IFN epsilon, kappa, omega and delta. However, all type I IFNs bind exclusively to the IFN alpha receptor (IFNAR). IFN alphas are produced by leukocytes in response to different stimuli whereas IFN beta is produced by most cell types except leukocytes. IFN beta has 30% amino-acid homology with IFN alpha but with higher binding affinity to IFNAR when compared to IFN alpha. IFN alpha and beta are used in the treatment of various human cancers and disease of viral origin. In a specific embodiment, the IFNα protein is IFNα8 having an amino acid sequence of SEQ ID NO: **69.** In a specific embodiment, the IFNβ protein has an amino acid sequence of SEQ ID NO: **73.**

In another embodiment, the biologically active molecule is granulocyte colony stimulating factor (GCSF). Granulocyte colony stimulating factor (GCSF) is a 20 KDa glycoprotein with a 174- or 177-amino acids single polypeptide chain. The shorter form possesses greater activity and stability than the longer isoform and is the basis for commercial pharmaceutical GCSF products. GCSF stimulates the proliferation of neutropenic progenitor cells and their differentiation into granulocytes, and also activates mature neutrophils. GCSF is most frequently used in the treatment of chemotherapy-induced neutropenia. In a specific embodiment, the GCSF protein has an amino acid sequence of SEQ ID NO: **71.**

### 2. Compositions

In certain embodiments, the present invention relates to compositions, including pharmaceutical compositions, comprising the fusion protein and a pharmaceutically acceptable carrier or excipient.

Pharmaceutical compositions can be prepared by mixing the fusion protein with optional pharmaceutically acceptable carriers. Pharmaceutically acceptable carriers include solvents, dispersion media, isotonic agents and the like. Examples of carriers include water, saline solutions or other buffers (such as phosphate, citrate buffers), oil, alcohol, proteins (such as serum albumin, gelatin), carbohydrates (such as monosaccharides, disaccharides, and other carbohydrates including glucose, sucrose, trehalose, mannose, mannitol, sorbitol or dextrins), gel, lipids, liposomes, stabilizers, preservatives, antioxidants including ascorbic acid and methionine, chelating agents such as EDTA; salt forming counter-ions such as sodium; non-ionic surfactants such as TWEEN™, PLURONICS™ or polyethylene glycol (PEG), or combinations thereof.

The pharmaceutical compositions can contain more than one active compound. For example, the formulation can contain one or more fusion protein and/or one or more additional beneficial compound(s). The active ingredients can be combined with the carrier in any convenient and practical manner, e.g., by admixture, solution, suspension, emulsification, encapsulation, absorption and the like, and can be made in formulations such as powder (including lyophilized powder), suspensions that are suitable for injections, infusion, or the like. Sustained-release preparations can also be prepared.

In certain embodiments, the pharmaceutical compositions contains the fusion protein for human use. The pharmaceutical compositions can be prepared in an appropriate buffer including, but not limited to, citrate, phosphate, Tris, BIS-Tris, etc. at an appropriate pH and can also contain excipients such as sugars (50 mM to 500 mM of sucrose, trehalose, mannitol, or mixtures thereof), surfactants (e.g., 0.025% - 0.5% of Tween 20 or Tween 80), and/or other reagents. The formulation can be prepared to contain various amounts of fusion protein. In general, formulations for administration to a subject contain between about 0.1 mg/mL to about 200 mg/mL. In certain embodiments, the formulations can contain between about 0.5 mg/mL to about 50 mg/mL; between about 1.0 mg/mL to about 50 mg/mL; between about 1 mg/mL to about 25 mg/mL; or between about 10 mg/mL to about 25 mg/mL of fusion protein. In specific embodiments, the formulations contain about 1.0 mg/mL, about 5.0 mg/mL, about 10.0 mg/mL, or about 25.0 mg/mL of fusion protein.

### 3. Methods

Another aspect of the present invention relates to methods for making a fusion protein and compositions thereof.

### a. Producing the Fusion Protein

The method for making the fusion protein comprises (i) providing a bioactive molecule and an sFc fragment comprising a hinge region, (ii) modifying the hinge region to prevent it from forming a disulfide bond, and (iii) linking the bioactive molecule directly or indirectly to the scFc through the mutated hinge region to form the fusion protein, hybrid, conjugate, or composition thereof. The present disclosure also describes a method for purifying the fusion protein, comprising (i) providing a fusion protein, and (ii) purifying the fusion protein by Protein A or Protein G-based chromatography media.

The fusion protein may alternatively be expressed by well known molecular biology techniques. Any standard manual on molecular cloning technology provides detailed protocols to produce the fusion protein of the invention by expression of recombinant DNA and RNA. To construct a gene expressing a fusion protein of this invention, the amino acid sequence is reverse translated into a nucleic acid sequence, preferably using optimized codons for the organism in which the gene will be expressed. Next, a gene encoding the peptide or protein is made, typically by synthesizing overlapping oligonucleotides which encode the fusion protein and necessary regulatory elements. The synthetic gene is assembled and inserted into the desired expression vector. The synthetic nucleic acid sequences encompassed by this invention include those which encode the fusion protein of the invention, and nucleic acid constructs characterized by changes in the non-coding sequences that do not alter the biological activity of the molecule encoded thereby. The synthetic gene is inserted into a suitable cloning vector and recombinants are obtained and characterized. The fusion protein is expressed under conditions appropriate for the selected expression system and host. The fusion protein is purified by an affinity column of Protein A or Protein G (e.g., SoftMax®, AcroSep®, Sera-Mag®, or Sepharose®).

The fusion protein of the present invention can be produced in mammalian cells. Examples of mammalian cells include monkey COS cells, CHO cells, human kidney 293 cells, human epidermal A431 cells, human Colo205 cells, 3T3 cells, CV-1 cells, other transformed primate cell lines, normal diploid cells, cell strains derived from in vitro culture of primary tissue, primary explants, HeLa cells, mouse L cells, BHK, HL-60, U937, HaK or Jurkat cells.

The invention also describes a method for producing a single chain Fc (sFc) region of an immunoglobulin G, comprising mutating, substituting, or deleting the Cys in a hinge region of Fc of IgG In one embodiment, the Cys is substituted with Ser, Gly, The, Ala, Val, Leu, Ile, or Met. In another embodiment, the Cys is deleted. In an additional embodiment, a fragment of the hinge is deleted.

The invention provides a method for producing a fusion protein in a mammalian cell comprising: (a) providing a bioactive molecule and an IgG sFc fragment comprising a hinge region, wherein the bioactive molecule selected from the group consisting of erythropoietin, Factor IX, IFNα, GCSF, and IFNβ (b) mutating the hinge region of the sFc fragment by amino acid substitution and/or deletion to form a mutated hinge region of a mutated Fc without disulfide bond formation, and (c) combining the bioactive molecule and the mutated sFc fragment through the mutated hinge region, wherein the mutated hinge region comprises an amino acid sequence selected from the group consisting of SEQ ID NOs: 23-60, and wherein the fusion protein is a monomer that is incapable of forming a dimer.

### b. Using the Fusion Protein

The fusion protein of the invention can be administered intravenously, subcutaneously, intra-muscularly, or via any mucosal surface, e.g., orally, sublingually, buccally, sublingually, nasally, rectally, vaginally, or via pulmonary route.

The dose of the fusion protein of the invention will vary depending upon the subject and the particular mode of administration. The dosage required will vary according to a number of factors known to those skilled in the art, including, but not limited to, the fusion protein, the species of the subject and, the size of the subject. Dosage may range from 0.1

to 100,000 $\mu$g/kg body weight. The fusion protein can be administered in a single dose, in multiple doses throughout a 24-hour period, or by continuous infusion. The fusion protein can be administered continuously or at specific schedule. The effective doses may be extrapolated from dose-response curves obtained from animal models.

Additional specific embodiments of the present invention include, but are not limited to the following examples.

## EXAMPLE 1

**ERYTHROPOIETIN SINGLE CHAIN FC FUSION PROTEIN (EPO-sFc)**

### 1. Fusion Protein

In this example, a fusion protein was prepared having a structure of formula 1 discussed above:

$$(B)\text{-}(Hinge)\text{-}(C_H2\text{-}C_H3)$$

wherein:

the bioactive molecule (B) is erythropoietin (EPO) protein (SEQ ID NO: **65);**
the hinge region (Hinge) is a mutated IgG1 hinge (SEQ ID NO: **27);**
and ($C_H2$-$C_H3$) is a $C_H2$-$C_H3$ of IgG1 (SEQ ID NO: **62).**

The full-length amino acid sequence of the EPO-sFc fusion protein is shown in the Sequence Listing as SEQ ID NO: **66.**

### 2. Expression Vector

The EPO-sFc was produced using a DNA expression vector. The DNA fragment of the erythropoietin single chain Fc fusion protein (EPO-sFc) was assembled using overlapping primers by the method of assembly polymerase chain reaction (PCR). The assembled EPO-sFc fragment was then ligated into PacI and EcoRV sites of pZD vector (pcDNA3.1Neo, Invitrogen, Carlsbad, CA, cat. no.V790-20 with dhfr gene) to obtain pZD/EPO-sFc as shown in **Figure 2** and then transformed into *E. coli.* The expression vector construct contained the zeocin-resistance gene as a selection marker.

## EXAMPLE 2

**ESTABLISHMENT OF STABLE RECOMBINANT CELL LINES PRODUCING EPO-sFc**

CHO$_{dhfr-}$ cells were trypsinized and resuspended at a concentration of $3\times10^6$ cells/mL in CP-T buffer (Cyto pluse Cat. CP-T). 0.2 mL of cell suspension ($6\times10^5$ cells) was transfected with 10 $\mu$g of plasmid pZD/EPO-sFc by electroporation (PA4000 PulseAgile® electroporator, Cyto Pulse Sciences). After 48 hrs of growth in non-selective medium, the transfectants were incubated in the selective complete medium containing IMDM, 10% fetal bovine serum, Zeocin (Invitrogen Cat. 1486406) and 5 nM MTX (Sigma Cat. BCL5707V) to obtain high yield clone zE93. The expression of the secreted fusion protein in the culture medium was detected and quantified by Q-ELISA (Quantikine® IVD® Epo ELISA kit).

The original zE93 cells were cultivated in a 10-cm dish containing IMDM supplemented with 10% FBS, zeocin, and 0.1 $\mu$M MTX. Cells were maintained in a 37°C humidified 95% air/5% CO$_2$ incubator (Model 3326, Forma scientific). In order to adapt the cells in serum-free culture medium, the medium was changed from IMDM to JRH serum-free medium supplemented with 5% FBS, zeocin, and 0.1 $\mu$M MTX. When cells became stable, the cells were detached from 10-cm dish by trypsinization and then transferred to spinner flasks containing 50 mL JRH serum-free medium supplemented with the same percentage of FBS. When confluency reach 90% in 3-5 days, the cells were subcultured into spinner flask containing JRH serum-free medium supplemented with a lower percentage of FBS. Cells were adapted into lower serum conditions by stepwise decreasing the FBS percentage from 10% to 0% in spinner flasks.

The concentration of EPO-sFc fusion protein in serum samples were quantified by Quantikine® IVD® Epo ELISA kit (R&D Systems Inc., CN: DEP00). The serum dilution-fold was optimized and the plate layout for standards, controls, and specimens were determined before performing formal assays using the fusion protein. Absorbance at wavelength 450 nm and 600 nm was acquired by SoftMax® Pro 5 software.

High-yield clones were successfully obtained by selection, limiting dilution and stepwise MTX challenges to produce finally the fusion protein comprising the recombinant EPO linked to single chain Fc (i.e., EPO-sFc). The resulting fusion protein was purified for further *in vitro* or *in vivo* biological activity assays and pharmacokinetics studies.

### EXAMPLE 3

**CHROMATOGRAPHIC PURIFICATION OF EPO-sFc**

Both Protein A based resin (MabSelect SuRe™) and DEAE resin (DEAE FF anion exchange column) were used to purify EPO-sFc. After purification, the corresponding recovery rate was analyzed by quantitative ELISA, and the respective purity by SDS-PAGE. Detailed purification processes for EPO-sFc are described below.

#### 1. MabSelect SuRe™ purification

Culture medium from the high yield EPO-sFc producing cell line was applied to a Protein A based MabSelect SuRe™ column (GE; Cat. no.: 11-0034-93) with a loading ratio at about 4.8 mg for 1 mL resin. After 2 washing steps, the fusion protein was eluted with pH 3.0 elution buffer and the eluate was thereafter neutralized to pH 8.8. The MabSelect SuRe™ eluate was analyzed by Q-ELISA (Quantikine® IVD® EPO ELISA kit) to determine the quantity and recovery rate. The recovery rate was 65.8 %, indicative of an efficient binding and purification of the designed EPO-sFc by MabSelect SuRe™ Protein A column **(Table 3).**

#### 2. DEAE FF purification

Before loading to DEAE FF 1 mL Hitrap column, the buffer was exchanged to DEAE FF equilibrium buffer. Thereafter, the EPO-sFc containing cell medium prepared from high yield cell line as described Example 2 was loaded to DEAE FF 1 mL Hitrap column with a loading ratio at about 5.92 mg for 1 mL resin. After acidic wash step, the main peak was eluted by 40 mM Tris buffer containing 130mM NaCl, pH 8.0. DEAE FF eluate was then analyzed by Q-ELISA (Quantikine® IVD® EPO ELISA kit). The overall recovery rate was 15.48% **(Table 3).**

#### 3. Results

**Table 3** reports information pertaining to the EPO-sFc fusion protein purified with MabSelect SuRe™ and DEAE FF, respectively. The table shows that MabSelect SuRe™ yields high purity EPO-sFc efficiently by a single purification step.

**Figure 4** shows the EPO-sFc fusion protein produced using the methods discussed above revealed a major band by the SDS-PAGE (Lanes 1, 2, and 3). The MW of EPO-sFc was between 50-70 KDa with high content of glycosylation which was shown in a duplicated SDS-PAGE gel by Periodic Acid-Schiff (PAS) staining method.

Sialic acid was one of the important components for EPO-sFc to affect its half-life in body circulation. The MW of EPO-sFc was between 50-70 KDa with high content of glycosylation. According to **Table 3,** EPO-sFc was purified by different chromatographies including MabSelect SuRe™ and DEAE FF, respectively. MabSelect SuRe™ could yield high purity EPO-sFc efficiently by a single purification step. The DEAE FF anion exchange resin would provide further polish in purification to remove low sialic acid isoforms of EPO-sFc which might affect the half-life of EPO-sFc.

### EXAMPLE 4

**PHARMACOKINETIC STUDY FOR EPO-sFc**

Ten rats (body weights ranging from 276-300g rats) were purchased from BioLASCO Taiwan Co., Ltd. All rats were quarantined and acclimatized for four days prior to the initiation of the pharmacokinetic (PK) studies. The rats were divided into three testing groups, for the PK studies: (1) EPO-sFc purified by DEAE; (2) EPO-sFc purified by Protein A; and (3) original recombinant human EPO (EPREX®). The rats of groups (1) and (2) were dosed at 16.8 µg/kg and the rats of group (3) were dosed at 3.5 µg/kg. The proteins were dosed via subcutaneous (S.C.) administration. The rats were grouped and labeled with fur dye. Both reference protein (EPREX®, 3.5 µg/mL) and test fusion proteins EPO-sFc (DEAE resin) and EPO-sFc-(Protein A resin) (at 16.8µg/mL) were prepared with fresh sample diluent (0.25% bovine serum albumin (AppliChem, CN: A0850,0250)) in saline for injection. All injections were administered to the rats via the site of dorsal neck for S.C. route.

Blood samples were collected at 0.5, 1, 2, 5, 8, 12, 24, 48, 72, 96, 120, and 144 hours after injection and then centrifuged at 3,000 rpm for 20 minutes. The supernatant was stored at -70°C.

The EPO concentrations in serum samples were quantified by Quantikine® IVD® EPO ELISA kit (R&D Systems Inc., CN: DEP00). Before performing the assay, the serum dilution-fold was optimized and the plate was laid out for standards, controls, and specimens. The absorbances at wavelength 450 nm and 600 nm were acquired by SoftMax® Pro 5 software. The Cmax, Tmax, AUC values, and the elimination phase half-life ($T_{1/2}$) from the EPO concentrations in serum were calculated by PK Solutions 2.0™ software.

The subcutaneous (S.C.) pharmacokinetic profiles of EPO-sFc purified from DEAE FF and EPREX® in rats after administration with single dose are shown in **Figure 6** and the mean pharmacokinetic features are shown in **Table 5.** The half-life of EPO-sFc-DEAE was 22.3±0.38 (hrs). In contrast, the half-life of EPREX® was only 6.182±0.675 (hrs). The AUCs of EPO-sFc (DEAE) and EPREX® were 327.4±15.13 and 161.5±23.64 (ng-hr/mL), respectively. The Tmax of EPO-sFc (DEAE) and EPREX® was 18±0.00 and 9.33±2.31 (hr), respectively.

The half-life of EPO-sFc was shown to be prolonged for up to 3 fold when compared to the original EPO (EPREX®) product. The EPO-sFc of this instant invention would therefore allow for use in chronic kidney disease (CKD) or cancer patients to decrease the injection frequency and improve patient life quality.

## EXAMPLE 5

### BIOLOGICAL ACTIVITY ASSAY FOR EPO-sFc

Eight female BALB/c mice (8 weeks old) were divided into two groups for determining the biological activity of EPO. One group was dosed with the reference drug (EPREX®) and other group was dosed with the EPO-sFc fusion protein of the present disclosure (EPO-sFc). Reference drug (EPREX®, Johnson and Johnson) was purchased and freshly prepared at the concentrations of 336 μg/mL (equal to 40 IU/mL). The EPO-sFc fusion protein produced according to Example 3 was freshly prepared at the concentrations of 0.336 ng/mL (equivalent molar to 40 IU/mL of EPREX®). Each mouse was subcutaneously administered with 0.5 ml of EPREX® or EPO-sFc fusion protein on day 1, and then blood collection was performed on days 4 to 9, 11, and 13.

The number of reticulocytes is a good indicator of biological activity of erythropoietin as it represents recent production and allows for the determination of reticulocyte count and erythropoietin potency. The number of reticulocytes was determined by FACS. 1.0 mL of PBS and 5.0 μL of whole blood were added to a polystyrene tube for unstained sample. 1.0 mL of Thiazole Orange (BD Retic-Count™, cn:349204) and 5.0μL of whole blood were added to a polystyrene tube for stained sample. Both tubes were incubated in the dark at room temperature for 30 min, and then analyzed within 3.5 hours after incubation. The samples were gently mixed immediately prior to analysis. The reticulocytes was determined by flow-cytometry (BD FACSCalibur™) and analyzed by CellQuest Pro™ software. The percentage of reticulocytes of each sample was calculated to evaluate the efficacy area under the curve (AUC) and maximal percentage of reticulocyte ($RET_{max}$) by PK solutions 2.0 software (Summit, Montrose, CO, USA).

The reticulocyte counts were used to compare the activity of reference drug and EPO-sFc fusion protein by measuring AUEC and $RET_{max}$ **(Table 7).** The AUEC of 0 to 13 hours was 88.69 and 91.03 ng•hr/ml for EPREX® and EPO-sFc fusion protein, respectively. In addition, the RETmax of EPREX® and EPO-sFc fusion protein was 11.12% and 10.48%, respectively. The results of AUEC and $RET_{max}$ suggests that the single chain Fc portion of the EPO-sFc fusion protein does not significantly interfere with the function of the EPO portion since the biological activity of EPO-sFc was comparable to EPREX® in this Example.

## EXAMPLE 6

### FACTOR IX SINGLE CHAIN FC FUSION PROTEIN (FIX-sFc)

### 1. Fusion Protein

In this example, a fusion protein was prepared having a structure of formula 1 discussed above:

$$(B)\text{-}(Hinge)\text{-}(C_H2\text{-}C_H3)$$

wherein:

the bioactive molecule (B) is Factor IX protein (FIX) (SEQ ID NO: **67);**
the hinge region (Hinge) is a mutated IgG1 hinge (SEQ ID NO: **23);** and
($C_H2$-$C_H3$) is a $C_H2$-$C_H3$ of IgG1 (SEQ ID NO: **61).**

The full-length amino acid sequence of the FIX-sFc fusion protein is shown in the Sequence Listing as SEQ ID NO: **68.**

## 2. Expression Vector

The FIX-sFc was produced using a DNA expression vector. The DNA fragment of the Factor IX was assembled using overlapping primers by the method of assembly PCR. The full-length gene of Factor IX single chain Fc fusion protein (FIX-sFc) was amplified from the reaction mixture, ligated into PacI and ApaI sites of pZD vector (pcDNA3.1Neo, Invitrogen, Carlsbad, CA, cat. no.V790-20 with dhfr gene) to obtain pZD/FIX-sFc as shown in **Figure 3** and then transformed into *E. coli*. The expression vector construct contained the zeocin-resistance gene as a selection marker.

## EXAMPLE 7

## ESTABLISHMENT OF STABLE RECOMBINANT CELL LINES PRODUCING

## FIX-sFc

$CHO_{dhfr-}$ cells were trypsinized and resuspended at a concentration of $3 \times 10^6$ cells/mL in CP-T buffer (Cyto pluse Cat. CP-T). Then 0.2 mL of the cell suspension ($6 \times 10^5$ cells) was transfected with 15 $\mu$g of plasmid pZD/FIX-sFc by electroporation for the expression of rhFIX-sFc (PA4000 PulseAgile® electroporator, Cyto Pulse Sciences). After 48 hrs of growth in non-selective medium, the transfectants were cultured under selective complete medium containing IMDM, 10% fetal bovine serum, Geneticin (Invitrogen Cat. 10131-027) and 5 nM MTX (Sigma Cat. M-8407) to obtain high yield clone N18. After cells achieving confluent in 96-well plates, the level of rhFIX-sFc was assessed by Q-ELISA and clones with high expression were transferred to 6-well plate. These clones were subcultured at $1 \times 10^5$ cells per well and allowed to grow for 7 days before determining FIX-sFc containing media titer by Q-ELISA.

The capture antibody (mouse Factor IX monoclonal antibody, Bioporto, Denmark, Cat. HYB 133-09) in 1:1000 (1 $\mu$g/mL) was prepared in carbonate/bicarbonate coating buffer for use. 100 $\mu$l of the diluted capture antibody was added into wells of ELISA plate and incubated at 4°C overnight. A detection antibody (Rabbit factor IX polyclonal Ab, Abcam, (U.K.), Cat. Ab23335) was diluted in PBST (PBS with 0.05% Tween 20) at 1:1,000 and added to ELISA plates. HRP conjugate antibody (Peroxidase-AffiniPure Goat Anti-Rabbit Ab, Jackson ImmunoResearch, (USA), Cat.111-035-144) and TMB Peroxidase Substrate (KPL, Cat.53-00-03) were used to produce color. Finally, 100 $\mu$l 1N $H_2SO_4$ was added to stop the reaction. The absorbances at wavelength 450 nm and 600 nm were acquired by SoftMax® Pro 5 software. The concentration of FIX-sFc were determined by an equation ($X = [a/(Y-Y_0)-1](1/b) \times X_0$), and the C initial value, AUC value and elimination phase half-life ($T_{1/2}$) were calculated for the concentration of FIX-sFc using PK solution 2.0™ software.

The original N18 clone was cultivated in a 10-cm dish containing IMDM supplemented with 5% FBS, zeocin, and 0.01 $\mu$M MTX. Cells were maintained in a 37°C humidified 95% air/8% $CO_2$ incubator (Model 3326, Forma scientific). In order for cells to adapt in serum-free medium, the culture medium was changed from IMDM to EX-CELL® 325 PF CHO Serum-Free Medium supplemented with 2% FBS, zeocin, and 0.02 $\mu$M MTX. When cells became stable, the cells were detached from 10-cm dish by trypsinization and then transferred to spinner flasks containing 50 mL EX-CELL® 325 PF CHO Serum-Free Medium supplemented with the same percentage of FBS. When cell confluence reached 90% in 3-5 days, the cells were subcultured into spinner flask containing EX-CELL® 325 PF CHO Serum-Free Medium supplemented with a lower percentage of FBS. Cells were adapted into lower serum conditions by stepwise decreasing the FBS percentage from 2% to 0% in spinner. To further increase rhFIX-sFc productivity and activity, the clone was retransfected with pZD/FIX-sFc. Cells from the high yield clone, N18-reZB, were subsequently sorted by FACS to separate therein the high yield cell group and grow under EX-CELL® 325 PF CHO Serum-Free Medium. Cells from the clone N18-reZB were tested by different MTX condition to select cells producing high yield of rhFIX-sFc.

After a round of selection, one high-yield clone, N18-reZB-sp4-sp5, was further selected by Q-ELISA MTX challenge. Subsequently, the serum-free clone was retransfected with pZD/FIX-sFc by sorting and MTX challenging and subjected to function selection leading to high-yield clones. N18-reZB-sp4-sp5 was obtained in this process. The accumulated titer in batch culture of N18-reZB-sp4-sp5 was about 53.4 $\mu$g/mL as determined by the Q-ELISA.

## EXAMPLE 8

## CHROMATOGRAPHIC PURIFICATION OF FIX-sFc

Both Protein A resin (MabSelect SuRe™) and IX Select resin were used to purify FIX-sFc. After purification, the corresponding recovery rate was analyzed by quantitative ELISA and the respective purity by SDS-PAGE. Detailed purification processes for FIX-sFc are described below.

## 1. MabSelect SuRe™ **purification**

FIX-sFc producing cell line culture medium was applied to a Protein A based MabSelect SuRe™ column with a loading ratio about 1.84 mg for 1 mL resin. After 2 washing steps, the fusion protein was eluted from the column by pH 3.0 buffer. MabSelect SuRe™ eluate was analyzed by Q-ELISA to determine the quantity and recovery rate. The FIX-sFc was captured by MabSelect SuRe™ efficiently and a single step purification already yielded a highly purified preparation as shown in Lane 2 of **Figure 5.**

## 2. IX Select purification

The IXSelect affinity (GE; no.: 17-5505-01) resin was packed in 1 mL column. The resin was coupled with monoclonal antibody directed against FIX. The range of loading pH was from 6.5-8.0. The FIX-sFc medium was loaded to IXSelect column without buffer exchange. The loading ratio was about 1.69 mg for 1 mL resin. After washing step to remove unbound materials, the main peak was eluted by 20 mM Tris containing 2 M $MgCl_2$, pH 7.4 buffer. Thereafter, IXSelect eluate was analyzed by Q-ELISA.

## 3. Results

**Table 4** reports information pertaining to the FIX-sFc fusion protein purified with MabSelect SuRe™ and IX Select affinity resin, respectively. The table shows that MabSelect SuRe™ and IX Select yield high purity FIX-sFc efficiently by a single purification step.

**Figure 5** shows the FIX-sFc fusion protein produced using the methods discussed above revealed a major band by the SDS-PAGE (Lanes 1 and 2). The MW of Factor IX-sFc was between 100-110 KDa with high content of glycosylation which was shown in a duplicated SDS-PAGE gel by Periodic Acid-Schiff (PAS) staining method. The recovery rate of MabSelect SuRe™ and IXSelect was 88.34% and 20.05%, respectively **(Table 4).** The Protein A based MabSelect SuRe™ column chromatography purified FIX-sFc efficiently by a single step with high yield and purity as shown in Figure 5 due to the unexpected Protein A/G binding property of the sFc from the designed fusion protein FIX-sFc.

## EXAMPLE 9

## PHARMACOKINETIC STUDY FOR FIX-sFc

Eight SD rats, weighing 315-375 g, were purchased from BioLASCO Taiwan Co., Ltd. The rats were randomly divided into two groups for pharmacokinetic (PK) studies: (1) recombinant FIX (BeneFIX®) as a reference drug; and (2) FIX-sFc of the present disclosure. The rats of group (1) and (2) were dosed at 1 mg/kg via tail vein on Day 0. After injection, the rats were bled at 0, 0.25, 4, 8, 24, 48, 72, 96 and 168 hours after injection according to the testing schedule. The coagulant free blood samples were placed at room temperature for at least 30 minutes with the serum isolated by centrifugation at 3,000 rpm for 20 minutes. The serum samples were aliquoted and stored at -70°C until analysis.

The diluted capture antibody (mouse Factor IX monoclonal antibody, Bioporto, Denmark, Cat. HYB 133-09) in 1:1000 (1 µg/mL) was prepared using carbonate/bicarbonate coating buffer. A buffer capsule (Sigma, Cat. # C-3041) was dissolved in 100 mL $ddH_2O$ to yield 0.05 M buffer, pH 9.6, filtrated by 0.2 µm filter, and stored at 4 °C. 100 µl of the diluted captured antibody was added into wells of ELISA plate and incubated at 4 ° C overnight. The plates were blocked using 200 µl of blocking buffer (PBS, pH 7.2 containing 2.0 % BSA), and washed 3 times with 200 µl of PBS (PBS, pH 7.2). 100 µl of BeneFIX® (100 ng/mL, 50 ng/mL, 25 ng/mL, 12.5 ng/mL, 6.25 ng/mL, 3.125ng/mL, and 1.5625 ng/mL) and FIX-sFc at corresponding concentrations were added to each ELISA well, respectively, and incubated at 37 °C for 1 hour. The ELISA plates were then washed 3 times with 200 µl of PBST (PBS with 0.05% Tween 20). A detection antibody (Rabbit factor IX polyclonal Ab, Abeam, (U.K.), Cat. Ab23335) was diluted in PBST (PBS with 0.05% Tween 20) by 1:1,000 and added into ELISA plates. After incubation, the ELISA plates were washed 3 times with PBST. HRP conjugate antibody (Peroxidase-AffiniPure Goat Anti-Rabbit Ab, Jackson ImmunoResearch, (USA), Cat.111-035-144) and TMB Peroxidase Substrate (KPL, Cat.53-00-03) were added into the plates to produce color. Finally, 100 µl 1 N $H_2SO_4$ was used to stop the reaction. The absorbances of diluted serum at wavelength 450 nm and 600 nm were acquired by SoftMax® Pro 5 software. The concentration of FIX-sFc was determined using an equation $(X = [a/(Y-Y_0)-_1](1/b) \times X_0)$. The C initial value, AUC value and elimination phase half-life $(T_{1/2})$ were calculated by the concentration of FIX-sFc using PK solution 2.0™ software.

The intravenous (I.V.) pharmacokinetic profiles of FIX-sFc purified from MabSelect SuRe™ and BeneFIX® in rats after administration with a single dose are shown in **Figure 7** and the mean pharmacokinetic features are shown in **Table 6.** The half-life of FIX-sFc and BeneFIX® were 56.0 ± 13.1 and 11.91 ± 2.54 (hrs), respectively. The AUCs of FIX-sFc and BeneFIX® were 19080.3 ± 2606.4 and 46594.40 ± 3634.08 (ng-hr/mL), respectively. The C initial of FIX-sFc and BeneFIX® were 4668.0 ± 447.5 and 11790.98 ± 4898.85 (ng/mL), respectively.

The half-life of FIX-sFc of the present invention was about 56 hours in rats with I.V administration, which is 5X longer than that (11.91 ± 2.54 hrs) of BeneFIX®. The FIX-sFc is, therefore, a long-acting drug for hemophilia patients to decrease the injection frequency and improve patient's quality of life. The pharmacokinetic data indicated that the FIX-sFc of the invention might be bound to the Fc receptor, resulting in a lower C initial concentration and AUC and released slowly back into the blood stream, resulting in its longer half-life.

## EXAMPLE 10

### BIOLOGICAL ACTIVITY ASSAY OF FIX-sFc

Activated Partial Thromboplastin Time (APTT) test was used to determine the biological activity of FIX-sFc. Briefly, the reference drug (BeneFIX®, Wyeth) and FIX-sFc were freshly prepared and diluted in Factor IX deficient plasma (Haematologic Technologies, Inc) at concentrations of 10, 5, and 2.5 $\mu$g/ml (final volume = 500 $\mu$L). The samples (BeneFIX® or FIX-sFc) were then mixed with equal volume of Dade® Actin® FSL Reagent (Siemens Healthcare Diagnostics Products GmbH) and incubated at 37°C for 3 min. Finally, $CaCl_2$ was added to stop the clotting reaction and observe the clot formation. The clotting time and specific activity were recorded and calculated by parallel line method (PLA analysis).

As shown in **Table 8,** the clotting time was dependent on the concentration of BeneFIX® or FIX-sFc. The average APTT result was 30.1, 26.2, and 25.5 sec for the FIX-sFc at the concentration of 2.5, 5.0, and 10.0 $\mu$g/ml, respectively. As shown in Table 8, the FIX-sFc had a similar relative potency and specific activity compared to BeneFIX®. FIX-sFc maintained an equivalent biological activity to BeneFIX®, which suggests that the single chain Fc does not interfere with the function of FIX in the fusion protein.

## EXAMPLE 11

### INTERFERON ALPHA SINGLE CHAIN FC FUSION PROTEIN (IFN$\alpha$-sFc)

### 1. Fusion Protein

In this example, a fusion protein comprising interferon alpha (IFN$\alpha$) was prepared and subsequently used in later examples. IFN$\alpha$ is a representative example of the large family of IFN$\alpha$ molecules.

Specifically, an IFN$\alpha$ single chain fusion protein (IFN$\alpha$-sFc) was prepared having a structure of formula 1 discussed above:

$$(B)\text{-}(Hinge)\text{-}(C_H2\text{-}C_H3)$$

wherein:

the bioactive molecule (B) is interferon alpha (IFN$\alpha$) protein (SEQ ID NO: **69);**
the hinge region (Hinge) is a mutated IgG1 hinge (SEQ ID NO: **23);** and
($C_H2$-$C_H3$) is a $C_H2$-$C_H3$ of IgG1 (SEQ ID NO: **62).**

The full-length amino acid sequence of the IFN$\alpha$-sFc fusion protein is shown in the Sequence Listing as SEQ ID NO: **70.**

### 2. Expression Vector

The IFN$\alpha$-sFc was produced using a DNA expression vector. The DNA fragment of IFN$\alpha$-sFc was assembled using overlapping primers by the method of assembly polymerase chain reaction (PCR). The assembled IFN$\alpha$-sFc fragment was then ligated into PacI and EcoRV sites of pZD vector (pcDNA3.1Neo, Invitrogen, Carlsbad, CA, cat. no.V790-20 with dhfr gene) to obtain pZD/IFN$\alpha$-sFc as shown in **Figure 8** and then transformed into *E. coli.* The expression vector construct contained the zeocin-resistance gene as a selected marker.

**EXAMPLE 12**

**ESTABLISHMENT OF STABLE RECOMBINANT CELL LINES PRODUCING**

**IFN$\alpha$-sFc**

CHO$_{dhfr-}$ cells were trypsinized and resuspended at a concentration of $3\times10^6$ cells/mL in CP-T buffer (Cyto pluse Cat. CP-T). 0.2 mL of cell suspension ($6\times10^5$ cells) was transfected with 10 $\mu$g of plasmid pZD/IFN$\alpha$-sFc by electroporation (PA4000 PulseAgile® electroporator, Cyto Pulse Sciences). After 48 hrs of growth in non-selective medium, the transfectants were incubated in the selective complete medium containing IMDM, 10% fetal bovine serum, Zeocin (Invitrogen Cat. 1486406) and 5 nM MTX (Sigma Cat. BCL5707V) to obtain high yield clone 22-123-327-117-Re117. The expression of the secreted fusion protein in the culture medium was detected and quantified by Q-ELISA.
The original 22-123-327-117-Re117 cells were cultivated in a 10-cm dish containing IMDM supplemented with 10% FBS, zeocin, and 0.1 $\mu$M MTX. Cells were maintained in a 37°C humidified 95% air/5% CO$_2$ incubator (Model 3326, Forma scientific). In order to adapt the cells in serum-free culture medium, the medium was changed from IMDM to JRH serum-free medium supplemented with 5% FBS, zeocin, and 0.1 $\mu$M MTX. When cells became stable, the cells were detached from 10-cm dish by trypsinization and then transferred to spinner flasks containing 50 mL JRH serum-free medium supplemented with the same percentage of FBS. When confluency reached 90% in 3-5 days, the cells were subcultured into spinner flask containing JRH serum-free medium supplemented with a lower percentage of FBS. Cells were adapted into lower serum conditions by stepwise decreasing the FBS percentage from 10% to 0% in spinner flasks.
The concentration of IFN$\alpha$-sFc fusion protein in serum samples was quantified by an in-house IFN$\alpha$-sFc ELISA kit. Absorbance at wavelength 450 nm and 600 nm was acquired by SoftMax® Pro 5 software. High-yield clones were successfully obtained by selection, limiting dilution and stepwise MTX challenges to produce finally the fusion protein comprising the recombinant IFN alpha8 linked to single chain Fc (i.e., IFN$\alpha$-sFc). The resulting fusion protein was purified for further *in vitro* or *in vivo* biological activity assays and pharmacokinetics studies.

**EXAMPLE 13**

**CHROMATOGRAPHIC PURIFICATION OF IFN$\alpha$-sFc**

Protein A based resin (MabSelect SuRe™) was used to purify IFN$\alpha$-sFc. After purification, the corresponding recovery rate was analyzed by quantitative ELISA, and the respective purity by SDS-PAGE. The detailed purification processes for IFN$\alpha$-sFc fusion protein is described below.

1. MabSelect SuRe™ **purification**

Culture medium from the high yield IFN$\alpha$-sFc producing cell line was applied to a Protein A based MabSelect SuRe™ column (GE; Cat. no.: 11-0034-93) with a loading ratio at about 4.6 mg for 1 mL resin. After 2 washing steps, the fusion protein was eluted with 0.1M Glycine pH3.0 elution buffer. The main peak is eluted by elution buffer. The MabSelect SuRe™ eluate was analyzed by Q-ELISA to determine the quantity and recovery rate.

**2. Results**

IFN$\alpha$-sFc was purified by MabSelect SuRe™ using different buffer conditions. The results showed that the 0.1 M Glycine pH3.0 elution buffer could purify IFN$\alpha$-sFc efficiently with high purity for further determination of the physical-chemical properties. The eluates of purified IFN$\alpha$-sFc samples were analyzed by SDS-PAGE as shown in **Figure 9** which revealed the purified IFN$\alpha$-sFc as a major band in all buffers evaluated. The stabilizing additives (sucrose or urea) were used in the elution buffer to prevent precipitation. Some low molecular weight impurities appeared when urea was used as an additive in the buffer. The purity of IFN$\alpha$-sFc in the glycine elution buffer having a sucrose additive was found to be > 95%.

**EXAMPLE 14**

**PHARMACOKINETIC STUDY FOR IFN$\alpha$-sFc**

Eight rats, weighing from 276-300g, were purchased from BioLASCO Taiwan Co., Ltd. All rats were quarantined and acclimatized for four days prior to the initiation of the pharmacokinetic (PK) studies. The rats were divided into four testing groups for the PK studies: (1) Pegasys® (pegylated IFNα), (2) IFNα-sFc of this disclosure, (3) Peg-Intron® (pegylated IFNα), and (4) Roferon-A® (recombinant IFNα). The rats were dosed at 310 pmol/kg, which was converted equivalently into 18.6 μg/kg for Pegasys®, 13.95 μg/kg for IFNα-sFc, 9.7 μg/kg for Peg-Intron® and 5.89 μg/kg for Roferon-A®. All articles were freshly prepared with fresh sample diluents, 0.2% bovine serum albumin (AppliChem, CN: A0850,0250) in phosphate-buffered saline. The rats were grouped and labeled with fur dye. All injections were administered to the rats via the site of dorsal neck for subcutaneous (S.C.) route.

Blood samples were collected at 0.5, 1, 2, 4, 6, 12, 24, 36, 48, 60, 72, 96, 120, 144, 192, 240, 288 and 336 hours after injection respectively and then centrifuged at 3,000 rpm for 20 minutes. The supernatants were stored at -70°C. Additional blood samples were collected and stored for the rats dosed with Roferon-A® at 0.08, 0.25, and 8 hours after injection.

The interferon concentration in serum samples was quantified by ELISA method. Before performing the assay, the serum dilution-fold was optimized and the plate was laid out for standards, controls, and specimens. The absorbances at wavelength 450 nm and 600 nm were acquired by SoftMax® Pro 5 software. The Cmax, Tmax, and AUC values and the elimination phase half-life ($T_{1/2}$) from the IFN concentrations in serum were calculated by PK Solutions 2.0™ software.

The subcutaneous (S.C.) pharmacokinetic profiles of the different treatment Groups after administration with single dose are shown in **Figure 10** with their mean pharmacokinetic features shown in **Table 9.** The half-life of Pegasys®, IFNα-sFc, Peg-Intron® and Roferon-A® was 23.2, 50.2, 20.8 and 0.73 hrs, respectively. The AUCs of Pegasys®, IFNα-sFc, Peg-Intron® and Roferon-A® were 64694.5, 60621.9, 5307.6 and 489.2 pM/h, respectively. The Tmax of Pegasys®, IFNα-sFc, Peg-Intron®, and Roferon-A® were 32.0, 32.0, 6.0 and 0.67 hr, respectively.

Roferon-A® is a first generation recombinant IFNα product. Pegasys® and Peg-Intron® are both second generation products of IFNα, with PEG (polyethylene glycol) coupled to the native IFNα. The inclusion of PEG has previously been shown to significantly prolong the half-life of IFNα compared to the non-pegylated form. The increase in half-life by PEG was also observed in this Example, as shown by the pharmacokinetic data reported in **Table 9** and **Figure 10** (*compare* the data for Pegasys® and Peg-Intron® *with* the data for Roferon-A®).

**Table 9** and **Figure 10** also show that the half-life of IFNα is prolonged even further, compared to Pegasys®, Peg-Intron®, and Roferon-A®, when it is present in a single chain Fc fusion protein of the present disclosure. Specifically, the half-life of IFNα-sFc is more than 2 times longer than the two PEGylated interferons (Pegasys® and Peg-Intron®) and more than 68 times longer than recombinant IFNα (Roferon-A®).

## EXAMPLE 15

### BIOLOGICAL ACTIVITY ASSAY FOR IFNα-sFc

Antiviral activity of the IFN was determined by a cytopathic effect (CPE) inhibition assay. In brief, the wells of 96-well plates were seeded with $1.0 \times 10^4$ A549 cells and incubated at 37°C in a 5% $CO_2$ incubator for 24 hours. When cell growth formed confluent monolayers, cells were treated with various concentrations and forms of IFNα. Five-fold serial dilutions of the IFNs were prepared starting at a concentration of 178 ng/mL. The final volume of IFN added to the wells was 100 μl. After 24 hours, the medium was removed, and the cells were infected with murine encephalomyocarditis virus (EMCV) at a titer of $6 \times 10^4$ PFU/mL and incubated for 24 hours. Cytopathic effect (CPE) was observed in virus control wells without any interferon. 20 μl of MTS solution was added to each well, and the plates were incubated at 37°C in a 5% $CO_2$ incubator for 3 hours. The stained cells were analyzed by spectrophotometry at OD 490 nm in an automated plate reader. All assays were performed in triplicate and the overall mean was calculated.

The percentage inhibition of viral CPE at each concentration of IFN was calculated using the formula below:

$$\text{"Inhibition (\%)} = (\text{OD test well - OD virus control}) / (\text{OD cell control - OD virus control)"}$$

A four-parameter logistic curve and a 50% viral replication inhibition dose were both calculated by Sigma Plot software.

In addition, the antiviral activity of IFNα-sFc (sFc fragment incapable of forming disulfide-bonded dimer) was compared to IFNα-Fc ( sFc fragment capable of forming disulfide-bonded dimer) and the results are shown in **Figure 11** and **Table 10**. The antiviral activity of IFN-α-sFc ($IC_{50}$ = 0.0061 nM) was 5.1-fold higher than IFN-α-Fc ($IC_{50}$ = 0.0313 nM) and 14.7-fold higher than Pegasys® ($IC_{50}$ = 0.0894).

### Summary

The results obtained in Examples 14 and 15 demonstrate that the IFNα single chain Fc fusion protein of this disclosure (IFNα-sFc) has improved biological properties and advantages compared to other IFNα products, including:

(1) reduction in production cost by increasing purification yield through Protein A chromatography, (2) enhancement in anti-viral activity, (3) longer serum half-life, resulting in a decrease in dosing frequency, and (4) reduction in renal clearance. Although the half-life of a traditional dimerized Fc fusion protein (IFNα-Fc) (sFc fragment capable of forming disulfide-bonded dimer) and Pegasys® were prolonged compared to Roferon-A®, their biological activities were lower than IFNα-sFc. The lower biological activities seen with IFNα-Fc and Pegasys® is likely caused, at least in part, by the steric interference of receptor binding with the larger fusion molecules (i.e., sFc fragment capable of forming a disulfide-bonded dimer in IFNα-Fc and the branched 40 kDa PEG chain in Pegasys®).

The results shown in **Figures 10-11** and **Tables 9-10** demonstrate that the single chain Fc modification of this disclosure produces an IFNα having a prolonged half-life and enhanced biological activity compared to other forms of IFNα. These results suggest that the modification of the present invention can be used to produce highly potent and efficient pharmaceutical compositions containing peptide and protein based treatments.

## EXAMPLE 16

**COMPARISON OF THE RESPECTIVE BINDING AFFINITIES OF IFNα-sFc ( SFC FRAGMENT CAPABLE OF FORMING DISULFIDE-BONDED DIMER) AND IFNα-Fc (DIMER) TO INTERFERON ALPHA RECEPTOR 1 (IFNAR1) AND INTERFERON ALPHA RECEPTOR 2 (IFNAR2)**

The binding affinities of the interferon fusion proteins were determined by Kinetic/Affinity assay.

Briefly, 5 μg/mL rhIFN alpha receptor 2 (IFNAR2) (Sino biotechnology, CN.: 10359-H08H) prepared in immobilization buffer (10 mM sodium acetate buffer, pH 4.0). The rhIFNAR2 ($R_L$ = 800 RU) was immobilized on a CM5 sensor chip (GE, CN.: BR-1003-99) through conventional amine coupling on a Surface Plasmon Resonance (SPR) machine (GE, Model: Biacore X100). Sample solutions containing 12.5 to 200 nM IFNα-sFc or IFNα-Fc were prepared by a 2-fold series dilution with running buffer (PBS with 0.005% Tween 20, pH 7.4). Solutions prepared in previous steps were diluted by 2-fold series with running buffer to prepare 6.25 to 100 nM IFNα-sFc or IFNα-Fc single injection solutions. Then, 200 nM IFN-alpha receptor 1 (IFNAR1) (Sino biotechnology, CN.: 13222-H08H) solution was mixed with equal volume of IFN single injection solution prepared in previous step to prepare IFN-IFNAR1 mixture. The concentration of IFNAR1 was 100 nM, while the concentration of IFNα-sFc or IFNα-Fc ranged from 6.25 nM to 100 nM. The equilibrium association constant (Ka) and equilibrium dissociation constant (Kd) values of IFNα-sFc or IFNα-Fc were analyzed by BIAevaluation software to calculate the binding constant (KD) values by the Kd and Ka.

As shown in **Figures 12a-12b,** IFNα-sFc--IFNAR1 and IFNα-Fc--IFNAR1 had similar binding profiles. Specifically, **Table 11** shows that the binding associations of the IFNs were relatively similar, with IFNα-sFc having a Ka of 1.4E+06 1/Ms and IFNα-Fc having a Ka of 3.3E+06 1/Ms, respectively. However, in the dissociation stage, IFNα-sFcIFNAR1 had a slower dissociation profile (Kd) than that of IFNα-Fc--IFNAR1. As shown in **Table 11,** the dissociation (Kd) of IFNα-sFc and IFNα-Fc from IFNAR1 was 5.6E-03 and 5.6E-02 1/s, respectively. The affinity of interaction (KD) of IFNα-sFc and IFNα-Fc to IFNAR was determined to be 4.0E-09 and 1.7E-08 nM, respectively, based on the association (Ka) and dissociation (Kd) profiles observed. Therefore, the affinity of IFNα-sFc was 4.25-fold higher than IFNα-Fc for forming the ternary complexes.

The binding affinity results observed in this Example further support the theory discussed in the preceding Example that IFNα-sFc has enhanced biological activities, compared to IFNα-Fc, is likely due to less steric interference in receptor binding for the sFc fragment incapable of forming disulfide-bonded dimer compared to the sFc fragment incapable of forming disulfide-bonded dimer dimer.

## EXAMPLE 17

**GRANULOCYTE-COLONY STIMULATING FACTOR SINGLE CHAIN FC FUSION PROTEIN (GCSF-sFc)**

### 1. Fusion Protein

In this example, a fusion protein was prepared having a structure of formula 1 discussed above:

(B)-(Hinge)-($C_H2$-$C_H3$)

wherein:

the bioactive molecule (B) is granulocyte-colony stimulating factor (GCSF) protein (SEQ ID NO: **71);**
the hinge region (Hinge) is a mutated IgG1 hinge (SEQ ID NO: **23);** and
($C_H2$-$C_H3$) is a $C_H2$-$C_H3$ of IgG1 (SEQ ID NO: **72).**

The full-length amino acid sequence of the GCSF-sFc fusion protein is shown in the Sequence Listing as SEQ ID NO: **72**.

## 2. Expression Vector

The GCSF-sFc was produced using a DNA expression vector. The DNA fragment of the granulocyte-colony stimulating factor single chain Fc fusion protein (GCSF-sFc) was assembled using overlapping primers by the method of assembly polymerase chain reaction (PCR). The assembled GCSF-sFc fragment was then ligated into PacI and EcoRV sites of pZD vector (pcDNA3.1Neo, Invitrogen, Carlsbad, CA, cat. no.V790-20 with dhfr gene) to obtain pZD-GCSF-sFc as shown in **Figure 13** and then transformed into *E. coli.* The expression vector construct contained the zeocin-resistance gene as a selection marker.

## EXAMPLE 18

## ESTABLISHMENT OF STABLE RECOMBINANT CELL LINES PRODUCING

## GCSF-sFc

$CHO_{dhfr-}$ cells were trypsinized and resuspended at a concentration of $3 \times 10^6$ cells/mL in CP-T buffer (Cyto pluse Cat. CP-T). 0.2 mL of cell suspension ($6 \times 10^5$ cells) was transfected with 10 $\mu$g of plasmid pZD-GCSF-sFc by electroporation (PA4000 PulseAgile® electroporator, Cyto Pulse Sciences). After 48 hrs of growth in non-selective medium, the transfectants were incubated in the selective complete medium containing IMDM, 10% fetal bovine serum, Zeocin (Invitrogen Cat. 1486406) and 5 nM MTX (Sigma Cat. BCL5707V) to obtain high yield clone zG3-17-41. The expression of the secreted fusion protein in the culture medium was detected and quantified by Q-ELISA.

The original zG3-17-41 cells were cultivated in a 10-cm dish containing IMDM supplemented with 10% FBS, zeocin, and 0.1 $\mu$M MTX. Cells were maintained in a 37°C humidified 95% air/5% $CO_2$ incubator (Model 3326, Forma scientific). In order to adapt the cells in serum-free culture medium, the medium was changed from IMDM to JRH serum-free medium supplemented with 5% FBS, zeocin, and 0.1 $\mu$M MTX. When cells became stable, the cells were detached from 10-cm dish by trypsinization and then transferred to spinner flasks containing 50 mL JRH serum-free medium supplemented with the same percentage of FBS. Cells were adapted into lower serum conditions by stepwise decreasing the FBS percentage from 10% to 0% in spinner flasks.

The concentration of GCSF-sFc fusion protein in serum samples was quantified by GCSF-sFc ELISA analysis. Absorbance at wavelength 450 nm and 600 nm was acquired by SoftMax® Pro 5 software. High-yield clones were successfully obtained by selection, limiting dilution and stepwise MTX challenges to produce finally the fusion protein comprising the recombinant GCSF linked to single chain Fc (i.e., GCSF-sFc). The resulting fusion protein was purified for further *in vitro* or *in vivo* biological activity assays and pharmacokinetics studies.

## EXAMPLE 19

## CHROMATOGRAPHIC PURIFICATION OF GCSF-sFc

Protein A based resin (MabSelect SuRe™) was used to purify GCSF-sFc. After purification, the corresponding recovery rate was analyzed by quantitative ELISA, and the respective purity by SDS-PAGE. The detailed purification process for GCSF-sFc is described below.

## MabSelect SuRe™ **purification**

Culture medium from the high yield GCSF-sFc producing cell line was applied to a Protein A based MabSelect SuRe™ column (GE; Cat. no.: 11-0034-93) with a loading ratio at about 3.4 mg for 1 mL resin. After 2 washing steps, the fusion protein was eluted with 0.1M Glycine pH3.0 elution buffer. The main peak eluted was analyzed by reverse phase HPLC (RP-HPLC). The MabSelect SuRe™ eluate was also analyzed by Q-ELISA to determine the quantity and recovery rate. The eluates of purified GCSF-sFc sample were also analyzed by SDS-PAGE. **Figure 14** reveals the GCSF-sFc fusion protein revealed as a major band by the SDS-PAGE. In summary, the GCSF-sFc could be captured by MabSelect SuRe™ efficiently.

## EXAMPLE 20

**PHARMACOKINETIC STUDY FOR GCSF-sFc**

Nine rats, weighing from 180-200g, were purchased from BioLASCO Taiwan Co., Ltd. All rats were quarantined and acclimatized for four days prior to the initiation of the pharmacokinetic (PK) studies. The rats were divided into three testing groups for the PK studies: (1) GCSF-sFc, (2) Lenograstim (Granocyte®), a recombinant GCSF, and (3) Peg-filgrastim (Neulasta®), a PEGylated form of recombinant human GCSF. The rats were dosed at 221.43 µg/Kg for GCSF-sFc and a molar equivalence of 100 µg/Kg for Lenograstim and Peg-filgrastim. All GCSF products were freshly prepared with sample diluents, 0.2% bovine serum albumin (AppliChem, CN: A0850,0250) in phosphate-buffered saline. The rats were grouped and labeled with fur dye. All injections were administered to the rats via the site of dorsal neck for subcutaneous (S.C.) route.

Blood samples were collected at 5 min, 0.5, 1, 2, 3, 8, 12, 24, 36, 48, 72, 96, 120, 144, and 168 hours after injection respectively and then centrifuged at 3,000 rpm for 20 minutes. The supernatants were stored at -70°C.

The GCSF concentrations in serum samples were quantified by ELISA method using paired antibodies (R&D System, CN: MAB214 and R&D System, CN: BAF214) to bind and detect GCSF. Before performing the assay, the serum dilution-fold was optimized and the plate was laid out for standards, controls, and specimens. The absorbances at wavelength 450 nm and 600 nm were acquired by SoftMax® Pro 5 software. The Cmax, Tmax, and AUC values and the elimination phase half-life ($T_{1/2}$) from the GCSF concentrations in serum were calculated by PK Solutions 2.0™ software.

The subcutaneous (S.C.) pharmacokinetic profiles of the respective GCSFs in rats after administration with single dose are shown in **Figure 15** with their mean pharmacokinetic features shown in **Table 12.** The half-life of GCSF-sFc, Lenograstim, and Peg-filgrastim was 17.16, 4.1 and 12.0 hrs, respectively. The Cmax of GCSF-sFc, Lenograstim, and Peg-filgrastim was 906.9, 156.4 and 480.8 ng/mL, respectively. The Tmax of GCSF-sFc, Lenograstim, and Peg-filgrastim was 48, 0.5 and 10.3 hrs, respectively. The AUC of GCSF-sFc, Lenograstim, and Peg-filgrastim was 50624, 790-1292, and 23202 ± 2921 ng-h/mL, respectively.

Lenograstim is a first generation recombinant GCSF product. Peg-filgrastim is a second generation product of GCSF coupled to PEG (polyethylene glycol). The inclusion of PEG has previously been shown to prolong the half-life of GCSF when compared to the first generation product (Lenograstim). The increase in GCSF half-life with PEG was also observed in this Example, as shown in the pharmacokinetics data reported in **Table 12** and **Figure 15** *(compare the data for Lenograstim with the data for Peg-filgrastim).*

**Table 12** and **Figure 15** also show that the half-life of GCSF is prolonged even further, compared to Lenograstim and Peg-filgrastim, when GCSF is present in a single chain Fc fusion protein of the present disclosure. Specifically, the half-life of GCSF-sFc is nearly one and a half times longer than PEGylated GCSF (Peg-filgrastim) and more than 4 times longer than recombinant GCSF (Lenograstim).

## EXAMPLE 21

**BIOLOGICAL ACTIVITY ASSAY FOR GCSF-sFc**

Neutropenic mice were used to analyze GCSFs potency in comparison to the native GCSF, i.e. Lenograstim (Granocyte®). In this study, 30 male BALB/cByJNarl mice aged 6 weeks were divided into six groups (i.e., five mice per group). All mice were treated with CPA (Cyclophosphamide monohydrate; Sigma, CN.: C7397) (100 mg/kg) on day 0. Group A as the control group only received a 0.9% NaCl/0.1% BSA solution on day 1. Three reference groups received Lenograstim (Chugai, CN.: N3L212) in a single shot (Groups B1 and B2) or four shots (Group B3) containing a dose of 51.02, 116.07 and 12.75 µM/kg, respectively. Two test groups received a single shot of GCSF-sFc containing a dose of 51.02 µM/kg (Group C1) and 116.07 µM/kg (Group C2). The groups that received a single shot (Groups A, B1, B2, C1, and C2) were injected on day 1 and the group receiving 4 shots (Group B3) was injected on days 1, 2, 3 and 4. All shots were administered via the route of subcutaneous injection. Blood samples were collected each day after administration and analyzed by Hematoanalyzer (HEMAVET 950 LV) until termination of the study. A blood sample was also taken 3 days prior to CPA treatment (day -3) for reference. The experimental design used in this study is summarized in **Table 13.**

**Figure 16** shows that pre-treating the mice with CPA caused neutropenia, i.e., a reduced the neutrophil counts in the blood of treated mice *(compare days -3 and 0 with day 1 in all samples)*. However, the neutropenia was found to be temporary and the level of neutrophils slowly returned to pre-CPA treatment levels after about 8 days in the control group *(see results for Group A)*. A single shot of Lenograstim at day 1, with either a mid-dose of 51.02 µM/Kg (Group B1) or a high-dose of 116.07 µM/Kg (Group B2), did not have any appreciable effect in increasing the neutrophil levels above those observed in the CPA control group during the observation period *(compare Groups B1 and B2 with Group*

A). Multiple administrations (4 shots) of Lenograstim following CPA treatment (Group B3) had some effect in increasing neutrophil levels compared to Groups A, B1 and B2 (see Group B3, days 3-5). However, the observed effect was minimal and short-lived because neutrophil levels were barely raised above the normal range and the levels quickly decreased to the normal levels following the multiple administration period (see Group B3, days 5 et seq).

In contrast, administration of a single administration of a mid-dose (51.02 $\mu$M/Kg) or high dose (116.07 $\mu$M/kg) of GCSF-sFc following CPA treatment efficiently raised the neutrophil levels significantly above the control group and all of the groups treated with Lenograstim (compare Groups C1 and C2 with Groups A, B1, B2, and B3).

Thus, the GCSF single chain Fc fusion protein of the present disclosure (GCSF-sFc) has a longer half-life and is more potent and efficient in enhancing neutrophil differentiation and proliferation in a neutropenic animal disease model compared to the native and PEGylated forms of GCSF.

## EXAMPLE 22

### INTERFERON BETA SINGLE CHAIN FC FUSION PROTEIN (IFNβ-sFc)

### 1. Fusion Protein

In this example, a fusion protein was prepared having a structure of formula 1 discussed above:

$$(B)\text{-}(Hinge)\text{-}(C_H2\text{-}C_H3)$$

wherein:

the bioactive molecule (B) is interferon beta (IFNβ) protein (SEQ ID NO: **73)**;
the hinge region (Hinge) is a mutated IgG1 hinge (SEQ ID NO: **23)**; and
($C_H2$-$C_H3$) is a $C_H2$-$C_H3$ of IgG1 (SEQ ID NO: **63)**.

The full-length amino acid sequence of the IFNβ-sFc is shown in the Sequence Listing as SEQ ID NO: **74.**

### 2. Expression Vector

The IFNβ-sFc was produced using a DNA expression vector. The DNA fragment of the IPNβ-sFc was assembled using overlapping primers by the method of assembly polymerase chain reaction (PCR). The assembled IFNβ-sFc fragment was then ligated into PacI and EcoRV sites of pZD vector (pcDNA3.1Neo, Invitrogen, Carlsbad, CA, cat. no.V790-20 with dhfr gene) to obtain pZD/IFNb-sFc as shown in **Figure 17** and then transformed into *E. coli.* The expression vector construct contained the zeocin-resistance gene as a selection marker.

## EXAMPLE 23

### ESTABLISHMENT OF STABLE RECOMBINANT CELL LINES PRODUCING

### IFNβ-sFc

CHO$_{dhfr-}$ cells were trypsinized and resuspended at a concentration of $3\times10^6$ cells/mL in CP-T buffer (Cyto pluse Cat. CP-T). 0.2 mL of cell suspension ($6\times10^5$ cells) was transfected with 10 $\mu$g of plasmid pZD-IFNβ-sFc by electroporation (PA4000 PulseAgile® electroporator, Cyto Pulse Sciences). After 48 hrs of growth in non-selective medium, the transfectants were incubated in the selective complete medium containing IMDM, 10% fetal bovine serum, Zeocin (Invitrogen Cat. 1486406) and 5 nM MTX (Sigma Cat. BCL5707V) to obtain a high yield clone zG3-17-41. The expression of the secreted fusion protein in the culture medium was detected and quantified by Q-ELISA.

The original Z-BsFc cells were cultivated in a 10-cm dish containing IMDM supplemented with 10% FBS, zeocin, and 0.1 $\mu$M MTX. Cells were maintained in a 37°C humidified 95% air/5% $CO_2$ incubator (Model 3326, Forma Scientific). In order to adapt the cells in serum-free culture medium, the medium was changed from IMDM to JRH serum-free medium supplemented with 5% FBS, zeocin, and 0.1 $\mu$M MTX. When cells became stable, the cells were detached from 10-cm dish by trypsinization and then transferred to spinner flasks containing 50 mL JRH serum-free medium supplemented with the same percentage of FBS. When confluency reached 90% in 3-5 days, cells were subcultured into spinner flask containing JRH serum-free medium supplemented with a lower percentage of FBS. Cells were adapted into lower serum conditions by stepwise decreasing the FBS percentage from 10% to 0% in spinner flasks.

The concentration of IFNβ-sFc fusion protein in serum samples were quantified by an in-house IPNβ-sFc ELISA kit. The serum dilution-fold was optimized and the plate layout for standards, controls, and specimens were determined before performing formal assays using the fusion protein. Absorbance at wavelength 450 nm and 600 nm was acquired by SoftMax® Pro 5 software. High-yield clones were successfully obtained by selection, limiting dilution and stepwise MTX challenges to produce finally the fusion protein comprising the recombinant IFN beta linked to single chain Fc (i.e., IFNβ-sFc). The resulting fusion protein was purified for further *in vitro* or *in vivo* biological activity assays and pharmacokinetics studies.

## EXAMPLE 24

### CHROMATOGRAPHIC PURIFICATION OF IFNβ-sFc

Protein A based resin (MabSelect SuRe™) was used to purify IFNβ-sFc. After purification, the corresponding recovery rate was analyzed by quantitative ELISA, and the respective purity by SDS-PAGE. The detailed purification process for IFNβ-sFc is described below.

### MabSelect SuRe™ purification

Culture medium from the high yield IFNβ-sFc producing cell line was applied to a Protein Abased MabSelect SuRe™ column (GE; Cat. no.: 11-0034-93) with a loading ratio of about 1.58 mg for 1 mL resin. After 2 washing steps, the fusion protein was eluted with 0.1M Glycine pH3.0 elution buffer. The protein that eluted in the main peak from the MabSelect SuRe™ column was analyzed and found to be relatively pure, based on the sharp peak observed by reverse phase HPLC (RP-HPLC) and the sharp band obtained by SDS-PAGE (data not shown). The MabSelect SuRe™ eluate was also analyzed by Q-ELISA to determine the quantity and recovery rate as shown in **Table 14.**

## EXAMPLE 25

### PHARMACOKINETIC STUDY FOR IFNβ-sFc

Eight Lewis rats, weighing from 200-230g, were purchased from BioLASCO Taiwan Co., Ltd. All rats were quarantined and acclimatized for four days prior to the initiation of the pharmacokinetic (PK) studies. The rats were divided into two testing groups for the PK studies: (1) natural fibroblast derived human IFNβ-1a (Rebif®) and (2) IFNβ-sFc. Rebif® and IFNβ-sFc were administered to the rats at a dose of 37 μg/Kg. All articles were freshly prepared with fresh sample diluents, 0.2% bovine serum albumin (AppliChem, CN: A0850,0250) in phosphate-buffered saline. The rats were grouped and labeled with fur dye. All injections were administered to the rats via the site of dorsal neck for subcutaneous (S.C.) route.

Blood samples were collected at 5 min, 0.5, 1, 4, 7, 12, 24, 36, 48, 72, 96, 120, 144, and 168 hours after injection respectively and then centrifuged at 3,000 rpm for 20 minutes. The supernatants were stored at -70°C.

The interferon concentrations in serum samples were quantified by VeriKine™ Human IFN Beta ELISA Kit (PBL assay science, CN.: 41410) for IFNβ. The absorbances at wavelength 450 nm and 600 nm were acquired by SoftMax® Pro 5 software. The Cmax, Tmax, AUC values, and the elimination phase half-life ($T_{1/2}$) from the interferon concentrations in serum were calculated by PK Solutions 2.0™ software.

The subcutaneous (S.C.) pharmacokinetic profiles of IFNβ in rats after administration with a single dose are shown in **Figure 18** and the mean pharmacokinetic features are reported in **Table 15.** The half-life of Rebif® and IFNβ-sFc was 18.92-23.57 and 31.89-37.7 hrs, respectively. The Cmax of Rebif® and IFNβ-sFc was 3.2±0.71 and 6.55±0.78 ng/mL, respectively. The Tmax of Rebif® and IFNβ-sFc was 12±0 and 9.55±3.61 hrs, respectively. The AUC of Rebif® and IFNβ-sFc was 115±18.38 and 273±2.83 ng-hr/mL, respectively.

The IFNβ-sFc of this invention exhibited improvement in half-life extension as shown in **Figure 18** and **Table 15** when compared to that of Rebif® (the native form of IFNβ).

## EXAMPLE 26

### BIOLOGICAL ACTIVITY ASSAY FOR IFNβ-sFc

IFNβ is an anti-inflammatory cytokine and serves as one of the major drugs for multiple sclerosis (MS) treatment. MS is the most common inflammatory disease of the central nervous system. The immune cells cross the blood brain barrier and attack myelin, leading to ineffective conduction of signals in nervous systems of MS patients. Injections of IFNβ drug several times per week is necessary to control the relapse of MS. The anti-inflammatory mechanism of IFNβ on MS has been reported to involve a shift in cytokine balance from Th1 to Th2 in the T-cell response against elements of the myelin sheath. In addition to the Th1 and Th2 groups, two other important pro-inflammatory cytokines, including osteopontin (OPN), have been found to play important roles in CNS inflammation in the pathogenesis of MS. IFNβ has been shown to inhibit the production of OPN in primary T cells derived from PBMC and the inhibition occurs at the CD4+ T-cell level.

In this study, the biological activity of IFNβ-sFc was evaluated in an osteopontin inhibition assay and its activity was compared to natural IFNβ-1a (Rebif®). Briefly, the osteopontin inhibition assay was carried out as follows: Human PBMCs were isolated from whole blood. For PBMC stimulation, 96-well flat-bottom tissue culture plate was pre-coated with 100 μl of anti-CD3 antibody at 1 μg/mL concentration per well and incubated overnight at 4°C. Each well was washed with 200 μl RPMI 1640 (with FBS), and seeded with $5 \times 10^4$ cells. Individual wells were treated with either 1 μg/mL anti-CD28 antibody, IFNβ-sFc (1.5 or 10 ng/mL), or Rebif® (1.5 or 10 ng/mL), and the plates were incubated for 48 hours at 37°C in 5% $CO_2$ humidified incubator. After incubation, the supernatant from each well was harvested and stored in -70°C freezer. The amount of the human Osteopontin (OPN) was measured by ELISA (R&D systems, CN.: DOST00).

The OPN concentration in each sample was calculated as follows: Separate standard curves were plotted with OPN on the x-axis and absorbance ($OD_{450}$-$OD_{570}$) on the y-axis and a fit line was drawn using a 4-parameter logistic equation. The OPN concentrations were then calculated using the following equation: $X = X0 \times \{[a/(Y-Y0)-1]^{\wedge}(1/b)\}$, as described in Chen M. et al. "Regulatory effects of IFN-β on production of osteopontin and IL-17 by CD4+ T Cells in MS" Eur. J. Immunol. 39, 2525-2536 (2009).

As shown in **Figure 19,** the single chain Fc fusion protein of the present disclosure (IFNβ-sFc) significantly inhibited the production of OPN in cells treated with 1.5 ng/mL (73.4±0.8%) and 10 ng/mL (79.5±3.6%) of the test article. Cells treated with the reference article (Rebif®) demonstrated a similar inhibition of OPN at 1.5 ng/mL (88.4±2.7%) and 10 ng/mL (81.3±13.3%). These results suggest that the inclusion of a single chain Fc does not significantly interfere with the ability of the IFNβ portion of the fusion protein to efficiently bind to its receptor since IFNβ-sFc has a comparable biological activity to that of the native IFNβ.

**TABLE 1**

| Examples of Mutated Hinge Regions | | |
|---|---|---|
| **Type of IgG** | **Sequence** | **SEQ ID NO** |
| Wild-type IgG1 | EPKSCDKTHTCPPCP | SEQ ID NO: 1 |
| Mutated IgG1 | EPKSXDKTHTXPPXP | SEQ ID NO: 2 |
| | EPKSXDKTHTXPP | SEQ ID NO: 3 |
| | EPKSXDKTHT | SEQ ID NO: 4 |
| | DKTHTXPPXP | SEQ ID NO: 5 |
| Wild-type IgG2 | ERKCCVECPPCP | SEQ ID NO: 6 |
| Mutated IgG2 | ERKXXVEXPPXP | SEQ ID NO: 7 |
| | ERKXXVEXPP | SEQ ID NO: 8 |
| | VEXPPXP | SEQ ID NO: 9 |
| Wild-type IgG3 | ELKTPLGDTTHTCPRCP | SEQ ID NO: 10 |
| Mutated IgG3 | ELKTPLGDTTHTXPRXP | SEQ ID NO: 11 |
| | ELKTPLGDTTHTXPR | SEQ ID NO: 12 |
| | ELKTPLGDTTHT | SEQ ID NO: 13 |
| Wild-type IgG3 | EPKSCDTPPPCPRCP | SEQ ID NO: 14 |
| Mutated IgG3 | EPKSXDTPPPXPRXP | SEQ ID NO: 15 |
| | EPKSXDTPPPXPR | SEQ ID NO: 16 |
| | EPKSXDTPPP | SEQ ID NO: 17 |
| | DTPPPXPRXP | SEQ ID NO: 18 |
| Wild-type IgG4 | ESKYGPPCPSCP | SEQ ID NO: 19 |

(continued)

| Examples of Mutated Hinge Regions | | |
|---|---|---|
| Type of IgG | Sequence | SEQ ID NO |
| Mutated IgG4 | EXKYGPPCPXCP | SEQ ID NO: 20 |
| | EXKYGPPCP | SEQ ID NO: 21 |
| | KYGPPCPXCP | SEQ ID NO: 22 |
| X : Ser, Gly, Thr, Ala, Val, Leu, Ile, Met, and/or deletion | | |

**TABLE 2**

| Examples of Amino Acid Sequences of Mutated Hinge Regions Derived from IgG1 | |
|---|---|
| Amino acid sequences [1] | SEQ ID NO |
| EPKSSDKTHTSPPSP | SEQ ID NO: 23 |
| EPKSSDKTHTSPP | SEQ ID NO: 24 |
| EPKSSDKTHTSPPP | SEQ ID NO: 25 |
| EPKSSDKTHT | SEQ ID NO: 26 |
| DKTHTSPPSP | SEQ ID NO: 27 |
| DKTHTSPP | SEQ ID NO: 28 |
| EPKSDKTHTPPP | SEQ ID NO: 29 |
| EPKSDKTHTSPPSP | SEQ ID NO: 30 |
| EPKSGDKTHTGPPGP | SEQ ID NO: 31 |
| EPKSGDKTHTGPP | SEQ ID NO: 32 |
| EPKSGDKTHTGPPP | SEQ ID NO: 33 |
| EPKSGDKTHT | SEQ ID NO: 34 |
| DKTHTGPPGP | SEQ ID NO: 35 |
| DKTHTGPP | SEQ ID NO: 36 |
| EPKSDKTHTGPPGP | SEQ ID NO: 37 |
| EPKSGDKTHTSPPSP | SEQ ID NO: 38 |
| EPKSGDKTHTGPPSP | SEQ ID NO: 39 |
| EPKSSDKTHTGPPGP | SEQ ID NO: 40 |
| EPKSSDKTHTGPPSP | SEQ ID NO: 41 |
| EPKSSDKTHTGPP | SEQ ID NO: 42 |
| EPKSGDKTHTSPP | SEQ ID NO: 43 |
| EPKSTDKTHTTPPTP | SEQ ID NO: 44 |
| EPKSTDKTHTTPP | SEQ ID NO: 45 |
| EPKSTDKTHTTPPP | SEQ ID NO: 46 |
| EPKSTDKTHT | SEQ ID NO: 47 |
| DKTHTTPPTP | SEQ ID NO: 48 |
| DKTHTTPP | SEQ ID NO: 49 |
| EPKSDKTHTTPPTP | SEQ ID NO: 50 |

(continued)

| Examples of Amino Acid Sequences of Mutated Hinge Regions Derived from IgG1 | |
|---|---|
| Amino acid sequences [1] | SEQ ID NO |
| EPKSSDKTHTTPPTP | SEQ ID NO: 51 |
| EPKSSDKTHTSPPTP | SEQ ID NO: 52 |
| EPKSADKTHTLPPMP | SEQ ID NO: 53 |
| EPKSVDKTHTLPPIP | SEQ ID NO: 54 |
| EPKSLDKTHTAPPAP | SEQ ID NO: 55 |
| EPKSVDKTHTAPP | SEQ ID NO: 56 |
| EPKSMDKTHTVPP | SEQ ID NO: 57 |
| EPKSIDKTHTLPP | SEQ ID NO: 58 |
| DKTHTAPPLP | SEQ ID NO: 59 |
| DKTHTVPPLP | SEQ ID NO: 60 |
| [1] Underlined residues represent sites of mutation in relation to the sequence of wild-type IgG | |

**TABLE 3**

| Purification of EPO-sFc by Protein A Resin (MabSelect SuRe™) and DEAE Resin (DEAE FF) Chromatography Columns | | | | | |
|---|---|---|---|---|---|
| Resin | Sample | Concentration (mg/mL) | Volume (mL) | Amount (mg) | Recovery rate (%) |
| **MabSelect SuRe™** | Medium loading | 0.096 | 50 | 4.8 | |
| | pH 3.0 elute | 0.355 | 8.8 | 3.124 | 65.8 |
| **DEAE FF** | EPO-sFc | 0.296 | 20 | 5.92 | |
| | NaCl eluate | 0.067 | 14 | 0.938 | 15.48 |

**TABLE 4**

| Purification of FIX-sFc by Protein A Resin (MabSelect SuRe™) and IXSelect Resin Chromatography Columns | | | | | |
|---|---|---|---|---|---|
| Resin | Sample | Concentration (mg/mL) | Volume (mL) | Amount (mg) | Recovery rate (%) |
| **MabSelect SuRe™** | Medium loading | 0.0188 | 98 | 1.8424 | 88.34 |
| | pH 3.0 elute | 0.217 | 7.5 | 1.6275 | |
| **IXSelect** | FIX-sFc | 0.0188 | 90 | 1.692 | 20.05 |
| | NaCl eluate | 0.0261 | 13 | 0.3393 | |

**TABLE 5**

| Pharmacokinetic Features of Recombinant EPO (EPREX®) and EPO-sFc Purified From DEAE FF | | |
|---|---|---|
| | Recombinant EPO (EPREX®) | EPO-sFc |
| **Half-life (hr)** | 6.182±0.675 | 22.3±0.38* |

(continued)

| Pharmacokinetic Features of Recombinant EPO (EPREX®) and EPO-sFc Purified From DEAE FF | | |
|---|---|---|
| | Recombinant EPO (EPREX®) | EPO-sFc |
| Cmax (ng/mL) | 6.47±1.07 | 7.2±0.71 |
| Tmax (hr) | 9.33±2.31 | 18±0.00 |
| AUC (0-t) | 161.5±23.64 | 327.4±15.13 |
| Values represent means ± S.D. (CV).<br>* compared with EPREX®, P<0.05 | | |

### TABLE 6

| Pharmacokinetic Features of Recombinant FIX (BeneFIX®) and FIX-sFc Purified From MabSelect SuRe™ | | |
|---|---|---|
| | Recombinant FIX (BeneFIX®) | FIX-sFc |
| Half-life (hr) | 11.91 ± 2.54 | 56.0 ± 13.1 |
| C initial (ng/mL) | 11790.98 ± 4898.85 | 4668.0 ± 447.5 |
| AUC (ng•hr/mL) | 46594.40 ± 3634.08 | 19080.3 ± 2606.4 |
| Values represent means ± S.D. (I.V) | | |

### TABLE 7

| Potency Comparison Between Recombinant EPO (EPREX®) and EPO-sFc | | |
|---|---|---|
| | Recombinant EPO (EPREX®) | EPO-sFc |
| $AUEC_{0-13}$ (ng•hr/mL) | 88.69 | 91.03 |
| $RET_{max}$ | 11.12% | 10.48% |

### TABLE 8

| Potency Comparison Between Recombinant FIX (BeneFIX®) and FIX-sFc | | | | | | | |
|---|---|---|---|---|---|---|---|
| | APTT test (sec) | | | | | | |
| Dose | Recombinant FIX (BeneFIX®) | | | FIX-sFc | | | |
| | No. 1 | No. 2 | Average | No. 1 | No. 2 | No. 3 | Average |
| 10.0 μg/mL | 25.0 | 25.6 | 25.3 | 23.5 | 26.6 | 26.3 | 25.5 |
| 5.0 μg/mL | 28.1 | 27.0 | 27.6 | 23.5 | 27.6 | 27.6 | 26.2 |
| 2.5 μg/mL | 30.4 | 29.2 | 29.8 | 30.1 | 29.9 | 30.2 | 30.1 |
| Specific activity (IU/mg) | 200 | | | 218 | | | |
| Relative potency [1] | 1 | | | 1.09 | | | |
| [1] Relative potency is the ratio of the specific activity of the test sample compared to the specific activity of Recombinant FIX (i.e., 200 IU/mg) | | | | | | | |

TABLE 9

| Pharmacokinetics Features of Interferons in Rats After Subcutaneous Administration | | | | |
|---|---|---|---|---|
| | Pegasys® (Pegylated-IFNα) | IFNα-sFc | PEG-Intron® (Pegylated-IFNα) | Roferon-A® (recombinant-IFNα) |
| Half-life (Hr) | 23.2 | 50.2 | 20.8 | 0.73 |
| Cmax (pM) | 820.6 | 677.6 | 182.1 | 213.0 |
| Tmax (Hr) | 320 | 32.0 | 6.0 | 0.67 |
| AUC (pM/h) | 64694.5 | 60621.9 | 5307.6 | 489.2 |
| CL/kg (mL/h/kg) | 160.8 | 390.7 | 1759.1 | 682.0 |
| Vd/kg (mL/kg) | 4.8 | 5.4 | 58.6 | 638.2 |

TABLE 10

| Antiviral Activity of Interferons in Rats After Subcutaneous Administration | | | |
|---|---|---|---|
| | IFNα-sFc [2] (Monomer) | IFNα-Fc [3] (Dimer) | Pegasys® (Pegylated-IFNα) |
| $IC_{50}$ (nM) | 0.0061 | 0.0313 | 0.0894 |
| Ratio [1] | 1 | 0.20 | 0.07 |

[1] The ratio is a comparison of the respective sample $IC_{50}$ with the $IC_{50}$ of IFNα-sFc.

[2] The term "monomer" reflects a sFc fragment incapable of forming a disulfide-bonded dimer

[3] The term "monomer" reflects a sFc fragment capable of forming a disulfide-bonded dimer

TABLE 11

| Binding Affinity Features of IFNα-sFc ( sFc fragment incapable of forming a disulfide-bonded dimer) and IFNα-Fc ( sFc fragment capable of forming a disulfide-bonded dimer) to IFNAR1 | | | |
|---|---|---|---|
| | Ka (1/Ms) | Kd (1/s) | KD (nM) |
| IFNα-sFc (Monomer) | 1.4E+06 | 5.6E-03 | 4.0E-09 |
| IFNα-Fc (Dimer) | 3.3E+06 | 5.6E-02 | 1.8E-08 |

TABLE 12

| Pharmacokinetics Features of GCSFs in Rats After Subcutaneous Administration | | | |
|---|---|---|---|
| | GCSF-sFc | Granocyte® (Lenograstim) | Neulasta® (Peg-filgrastim) |
| Tmax (hr) | 48 | 0.5 | 10.3 |
| Cmax (ng/mL) | 906.9 | 156.4 | 480.8 |
| Half-life (hr) | 17.16 | 4.1 | 12.0 |
| AUC (ng-h/mL) | 50624 | 790-1292 | 23202±2921 |

**TABLE 13**

| Experimental Setup for Biological Activity Assay for GCSF-sFc | | | | |
|---|---|---|---|---|
| Group [1] | Sample | Treatment [2] | # Shots | Dose (μM/kg) |
| A | Control | 0.9% NaCl/0.1% BSA | 1 | -- |
| B1 | Reference | Lenograstim | 1 | 51.02 |
| B2 | Reference | Lenograstim | 1 | 116.07 |
| B2 | Reference | Lenograstim | 4 | 12.75 |
| C1 | Test | GCSF-sFc | 1 | 51.02 |
| C2 | Test | GCSF-sFc | 1 | 116.07 |

[1] Each Group contained 5 neutropenic mice

[2] All Groups were pre-treated with CPA (Cyclophosphamide monohydrate) on Day 0. Administration of the treatment drug was given on Day 1 for the Groups that received a single shot, and on Days 1, 2, 3, and 4 for the Group that received 4 shots.

**TABLE 14**

| Recovery Rate of IFNβ-sFc Purified with Protein A Resin, Determined by ELISA | | | | |
|---|---|---|---|---|
| | Concentration (mg/mL) | Volume (mL) | Amount (mg) | Recovery (%) |
| **Medium loading** | 0.01 | 925.0 | 8.33 | -- |
| **Eluate** | 0.25 | 25.0 | 6.34 | 76.13 |

**TABLE 15**

| Pharmacokinetics Features of IFNβ in Rats After Subcutaneous Administration | | |
|---|---|---|
| | Rebif® (Natural IFNβ) | IFNβ-sFc |
| $T_{1/2}$ (hr) | 18.92-23.57 | 31.89-37.70 |
| $T_{max}$ (hr) | 12.0010 | 9.55±3.61 |
| $C_{max}$ (ng/mL) | 3.20±0.71 | 6.55±0.78 |
| AUC (ng-hr/mL) | 115±18.38 | 273 ±2.83 |
| Clearance (mL/hr/kg) | 326±53.74 | 136±1.41 |

**Claims**

1. A fusion protein produced in a mammalian cell comprising:

   a) a bioactive molecule selected from the group consisting of erythropoietin, Factor IX, IFNα, GCSF, and INFβ; and
   b) a single chain Fc (sFc) fragment of an IgG molecule having an amino acid sequence selected from the group consisting of SEQ ID NOs: 61, 62, 63, and 64,
   c) a hinge region between the bioactive molecule and the sFc fragment, wherein the hinge region is mutated and does not form disulfide bonds with another fusion protein or another immunoglobulin molecule, and wherein the hinge region comprises an amino acid sequence selected from the group consisting of SEQ ID NOs: 23-60.

2. The fusion protein according to claim 1, wherein the hinge region comprises an amino acid sequence of SEQ ID NO: 23 or 27.

3. The fusion protein according to claim 1, wherein the bioactive molecule has an amino acid sequence selected from the group consisting of SEQ ID NOs: 65, 67, 69, 71, and 73.

4. The fusion protein according to claim 1, wherein the amino acid sequence of the fusion protein is selected from the group consisting of SEQ ID NOs: 66, 68, 70, 72, and 74.

5. A pharmaceutical composition comprising the fusion protein according to claim 1 and a pharmaceutically acceptable carrier or excipient.

6. A method for producing a fusion protein in a mammalian cell comprising:

a) providing a bioactive molecule and an sFc fragment comprising a hinge region, wherein the bioactive molecule selected from the group consisting of erythropoietin, Factor IX, IFN$\alpha$, GCSF, and INF$\beta$;
b) mutating the hinge region of the sFc fragment by amino acid substitution and/or deletion to form a mutated hinge region of a mutated sFc fragment, and
c) combining the bioactive molecule and the mutated sFc fragment through the mutated hinge region,

wherein the mutated hinge region comprises an amino acid sequence selected from the group consisting of SEQ ID NOs: 23-60, and wherein the mutated hinge region does not form disulfide bonds with another fusion protein or another immunoglobulin molecule.

7. A fusion protein produced in a mammalian cell comprising:

a) a single chain Fc fragment (sFc) of an IgG molecule having an amino acid sequence selected from the group consisting of SEQ ID NOs: 61, 62, 63, and 64;
b) a hinge region comprising an amino acid sequence selected from the group consisting of SEQ ID NOs: 23-60; and
c) a bioactive molecule having an amino acid sequence selected from the group consisting of SEQ ID NOs: 65, 67, 69, 71, and 73.

8. The fusion protein according to claim 7, wherein the amino acid sequence of the hinge region is SEQ ID NO: 23 or 27.


**Patentansprüche**

1. Fusionsprotein, das in einer Säugetierzelle hergestellt ist, umfassend:

a) ein bioaktives Molekül, das aus der Gruppe bestehend aus Erythropoietin, Faktor IX, IFN$\alpha$, GCSF, und INFß ausgewählt ist; und
b) ein einkettiges Fc-(sFc)-Fragment eines IgG-Moleküls mit einer Aminosäuresequenz, die aus der Gruppe bestehend aus SEQ ID NOs: 61, 62, 63, und 64 ausgewählt ist,
c) eine Gelenkregion zwischen dem bioaktiven Molekül und dem sFc-Fragment, wobei die Gelenkregion mutiert ist und keine Disulfidbindungen mit einem anderen Fusionsprotein oder einem anderen Immunglobulinmolekül bildet, und wobei die Gelenkregion eine Aminosäuresequenz umfasst, die aus der Gruppe bestehend aus SEQ ID NOs: 23-60 ausgewählt ist.

2. Fusionsprotein nach Anspruch 1, wobei die Gelenkregion eine Aminosäuresequenz der SEQ ID NO: 23 oder 27 umfasst.

3. Fusionsprotein nach Anspruch 1, wobei das bioaktive Molekül eine Aminosäuresequenz aufweist, die aus der Gruppe bestehend aus SEQ ID NOs: 65, 67, 69, 71, und 73 ausgewählt ist.

4. Fusionsprotein nach Anspruch 1, wobei die Aminosäuresequenz des Fusionsproteins aus der Gruppe bestehend aus SEQ ID NOs: 66, 68, 70, 72, und 74 ausgewählt ist.

5. Pharmazeutische Zusammensetzung, umfassend das Fusionsprotein nach Anspruch 1 und einen pharmazeutisch akzeptablen Träger oder Hilfsstoff.

**6.** Verfahren zur Herstellung eines Fusionsproteins in einer Säugetierzelle, umfassend:

a) Bereitstellen eines bioaktiven Moleküls und eines sFc-Fragments, das eine Gelenkregion umfasst, wobei das bioaktive Molekül aus der Gruppe bestehend aus Erythropoietin, Faktor IX, IFN$\alpha$, GCSF, und INFß ausgewählt ist;
b) Mutieren der Gelenkregion des sFc-Fragments durch eine Aminosäuresubstitution und/oder Deletion, um eine mutierte Gelenkregion eines mutierten sFc-Fragments zu bilden, und
c) Kombinieren des bioaktiven Moleküls und des mutierten sFc-Fragments durch die mutierte Gelenkregion,

wobei die mutierte Gelenkregion eine Aminosäuresequenz umfasst, die aus der Gruppe bestehend aus SEQ ID NOs: 23-60 ausgewählt ist, und wobei die mutierte Gelenkregion keine Disulfidbindungen mit einem anderen Fusionsprotein oder einem anderen Immunglobulinmolekül bildet.

**7.** Fusionsprotein, das in einer Säugetierzelle hergestellt ist, umfassend:

a) ein einkettiges Fc-Fragment (sFc) eines IgG-Moleküls mit einer Aminosäuresequenz, die aus der Gruppe bestehend aus SEQ ID NOs: 61, 62, 63, und 64 ausgewählt ist;
b) eine Gelenkregion, die eine Aminosäuresequenz umfasst, die aus der Gruppe bestehend aus SEQ ID NOs: 23-60 ausgewählt ist; und
c) ein bioaktives Molekül mit einer Aminosäuresequenz, die aus der Gruppe bestehend aus SEQ ID NOs: 65, 67, 69, 71, und 73 ausgewählt ist.

**8.** Fusionsprotein nach Anspruch 7, wobei die Aminosäuresequenz der Gelenkregion SEQ ID NO: 23 oder 27 ist.

**Revendications**

**1.** Protéine de fusion produite dans une cellule de mammifère comprenant:

a) une molécule bioactive choisie dans le groupe consistant en l'érythropoïétine, le facteur IX, IFN$\alpha$, GCSF et INF$\beta$; et
b) un fragment Fc à chaîne unique (sFc) d'une molécule d'IgG ayant une séquence d'acides aminés choisie dans le groupe consistant en SEQ ID NO: 61, 62, 63 et 64,
c) une région charnière entre la molécule bioactive et le fragment sFc, dans laquelle la région charnière est mutée et ne forme pas de liaisons disulfure avec une autre protéine de fusion ou une autre molécule d'immunoglobuline, et dans laquelle la région charnière comprend une séquence d'acides aminés choisie dans le groupe consistant en SEQ ID NO: 23 à 60.

**2.** Protéine de fusion selon la revendication 1, dans laquelle la région charnière comprend une séquence d'acides aminés de SEQ ID NO: 23 ou 27.

**3.** Protéine de fusion selon la revendication 1, dans laquelle la molécule bioactive a une séquence d'acides aminés choisie dans le groupe consistant en SEQ ID NO: 65, 67, 69, 71 et 73.

**4.** Protéine de fusion selon la revendication 1, dans laquelle la séquence d'acides aminés de la protéine de fusion est choisie dans le groupe consistant en SEQ ID NO: 66, 68, 70, 72 et 74.

**5.** Composition pharmaceutique comprenant la protéine de fusion selon la revendication 1 et un véhicule ou excipient pharmaceutiquement acceptable.

**6.** Procédé pour produire une protéine de fusion dans une cellule de mammifère comprenant:

a) la fourniture d'une molécule bioactive et d'un fragment sFc comprenant une région charnière, dans lequel la molécule bioactive choisie dans le groupe consistant en l'érythropoïétine, le facteur IX, IFN$\alpha$, GCSF et INF$\beta$;
b) la mutation de la région charnière du fragment sFc par substitution et/ou délétion d'acides aminés pour former une région charnière mutée d'un fragment sFc muté, et
c) la combinaison de la molécule bioactive et du fragment sFc muté par l'intermédiaire de la région charnière mutée,

dans lequel la région charnière mutée comprend une séquence d'acides aminés choisie dans le groupe consistant en SEQ ID NO: 23 à 60, et dans lequel la région charnière mutée ne forme pas de liaisons disulfure avec une autre protéine de fusion ou une autre molécule d'immunoglobuline.

7. Protéine de fusion produite dans une cellule de mammifère comprenant:

a) un fragment Fc à chaîne unique (sFc) d'une molécule d'IgG ayant une séquence d'acides aminés choisie dans le groupe consistant en SEQ ID NO: 61, 62, 63 et 64;
b) une région charnière comprenant une séquence d'acides aminés choisie dans le groupe consistant en SEQ ID NO: 23 à 60; et
c) une molécule bioactive ayant une séquence d'acides aminés choisie dans le groupe consistant en SEQ ID NO: 65, 67, 69, 71 et 73.

8. Protéine de fusion selon la revendication 7, dans laquelle la séquence d'acides aminés de la région charnière est SEQ ID NO: 23 ou 27.

Fig. 1A | Biologically active molecule | Hinge | $C_H2 + C_H3$ |

Fig. 1B | Biologically active molecule | Linker | Hinge | $C_H2 + C_H3$ |

Fig. 1C | $C_H2 + C_H3$ | Hinge | Biologically active molecule |

Fig. 1D | $C_H2 + C_H3$ | Hinge | Linker | Biologically active molecule |

Fig. 2

Fig. 3

Lane M: MW Marker
Lane 1: Medium
Lane 2: Protein A eluate
Lane 3: DEAE eluate

Fig. 4

EP 3 307 304 B1

Lane M: Molecular Weight Marker
Lane 1: Benefix rFIX (1µg)
Lane 2: Purified FIX-sFc by
       MabSelect SuRe (0.5µg)

Fig. 5

EPO Pharmacokinetic study in rat

Fig. 6

EP 3 307 304 B1

Factor IX PK study in rat

Fig. 7

EP 3 307 304 B1

Fig. 8

Fig. 9

Fig. 10

Antiviral activity assay of IFNα

% of inhibition

IFNα Conc. (nM)

Pegasys®
IFNα-sFc
IFNα-Fc

Fig. 11

IFNα-sFc (6.25 ~100 nM) + IFNAR1 (100 nM)

Fig. 12a

IFNα-Fc (6.25 ~100 nM) + IFNAR1 (100 nM)

Fig. 12b

Fig. 13

EP 3 307 304 B1

Lane M: MW Marker
Lane 1: GCSF-sFc

Fig. 14

Fig. 15

Fig. 16

Fig. 17

Fig. 18

# Osteopontin Inhibition Assay measured in CD3⁺ cells in the presence of anti-CD28 antibody

| IFN beta-sFc(ng/ml) | 0 | 1.5 | 10 | 0 | 0 |
| Rebif® (ng/ml) | 0 | 0 | 0 | 1.5 | 10 |

Fig. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6086875 A, Blumberg R. S.
- US 5624821 A, Winter G. P.
- US 5116964 A, Capon D. J. and Lasky L . A.
- US 6485726 B, Blumberg R. S.
- US 6030613 A
- WO 03077834 A, Blumberg R. S.
- US 20030235536 A1, Blumberg R. S.
- WO 9404689 A, Pastan I. H.
- US 6797493 B, Sun L-H.
- US 8557232 B, Gillies S. D.
- US 7501494 B

### Non-patent literature cited in the description

- **JANEWAY et al.** Immunobiology. Garland Publishing, 2001
- **CAPON et al.** *Nature,* 1989, vol. 337, 525
- **CAPON D. J. et al.** Designing CD4 immunoadhesins for AIDS therapy. *Nature,* 1989, vol. 337, 525
- **GOEBL N.A. et al.** Neonatal Fc Receptor Mediates Internalization of Fc in Transfected Human Endothelial Cells. *Mol. Biol. Cell,* 2008, vol. 19 (12), 5490-5505
- **JUNGHANS R.P et al.** The protection receptor for IgG catabolism is the beta2-microglobulin-containing neonatal intestinal transport receptor. *Proc Natl Acad Sci USA.,* 1996, vol. 93 (11), 5512-5516
- **OBER R. J. et al.** Exocytosis of IgG as mediated by the receptor, FcRn: an analysis at the single-molecule level. *Proc Natl Acad Sci USA.,* 2004, vol. 101 (30), 11076-81
- **OBER R. J et al.** Visualizing the site and dynamics of IgG salvage by the MHC class I-related receptor, FcRn. *J Immunol.,* 2004, vol. 172 (4), 2021-9
- **OSBORN B. L. et al.** Pharmacokinetic and pharmacodynamic studies of a human serum albumin-interferon-alpha fusion protein in cynomolgus monkeys. *J Pharmacol Exp Ther,* 2002, vol. 303 (2), 540-8
- **PETERS R. T. et al.** Prolonged activity of factor IX as a monomeric Fc fusion protein. *Blood.,* 2010, vol. 115 (10), 2057-64
- **VACCARO C et al.** Engineering the Fc region of immunoglobulin G to modulate in vivo antibody levels. *Nat Biotechnol.,* 2005, vol. 23 (10), 1283-8
- **CHEN M. et al.** Regulatory effects of IFN-β on production of osteopontin and IL-17 by CD4+ T Cells in MS. *Eur. J. Immunol.,* 2009, vol. 39, 2525-2536